(19) Europäisches Patentamt
European Patent Office
Office européen des brevets

(11) Publication number: **0 181 568 A2**

# (12) EUROPEAN PATENT APPLICATION

(21) Application number: 85113830.5

(22) Date of filing: 30.10.85

(51) Int. Cl.[4]: **C 07 D 215/14**
C 07 D 213/30, C 07 D 213/79
C 07 C 43/23, C 07 C 49/76
C 07 D 215/22, C 07 D 213/84
C 07 C 65/21, C 07 C 69/76
C 07 C 121/75, C 07 C 47/575

(30) Priority: 30.10.84 US 666430
22.05.85 US 736795

(43) Date of publication of application:
21.05.86 Bulletin 86/21

(84) Designated Contracting States:
AT BE CH DE FR GB IT LI LU NL SE

(71) Applicant: USV PHARMACEUTICAL CORPORATION
1 Scarsdale Road
Tuckahoe New York(US)

(72) Inventor: Musser, John H.
1009 Millstream Road
Malvern Pennsylvania(US)

(72) Inventor: Chakraborty, Utpal Ranjan
51 Minuteman Circle
Orangeburg New York(US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

## (54) Anti-inflammatory/anti-allergic compounds.

(57) Compounds of the formula:

$Ar_1 - X - Ar - Z - (R)_{n'}$

and salts thereof,

wherein

$Ar_1$ is a nitrogen, sulfur, oxygen heterocyclic ring or aromatic ring;

Ar is a phenyl ring or a nitrogen, oxygen or sulfur heterocyclic ring;

Ar and $Ar_1$ may be fully substituted or less than fully substituted with H, $CH_3$, lower alkyl, aryl, aralkyl, halo, hydroxy, lower alkoxy, $CF_3$, carboxy, alkylcarboxy, arylcarboxy, alkylcarbalkoxy, alkanoyl, formyl, oxo, nitrilo, amino, aminoalkyl, alkylamine, carboxamide, aryloxy, nitro, sulfonyl, sulfonamide, thio, or alkylthio;

$X = -O(CHR_1)_n-$, $-S(CHR_1)_n-$, $-NR_2(CHR_1)_n$-alkylene
||
$(O)_{n''}$

of up to 2 carbon atoms in the principal chain and up to a total of 4 carbon atoms,

$-C(R_1)=C(R_1)-$, $-C{\equiv}C-$,

$-CH(CHR_1)_n-$, $-CH=N-$, $-C-O-$,
|　　　　　　　　　||
OH　　　　　　　　O

$-C-S-$, or $-C-N(R_1)-$;
||　　　　　||
O　　　　　O

Z is an alkylene chain containing up to 10 carbon atoms in the principal chain and a total of up to 12 carbon atoms and from 0 to 2 double bonds and the said alkylene chain may be attached to Ar through an oxygen, sulfur or amino nitrogen atom, and when n'=2, one of the R substituents may be halogen on an omega carbon of the alkylene chain Z;

when n'=1, R is a substituent attached to one of the carbon atoms of Z selected from the group consisting of =O, $OR_3$, $SR_3$, $N(R_2)_2$ and $R_1$, $-COR_4$ and when n'=2 one R is previously defined and the additional R is a substituent attached to one of the carbon atoms of Z selected from the

./...

EP 0 181 568 A2

group consisting of $=O$, $OR_3$, $SR_3$, $N(R_2)_2$, $-COR_4$, lactone and halo;

$R_1$ is H or $CH_3$;

$R_2$ is H, lower alkyl, aryl or aralkyl;

$R_3$ is H, lower alkyl, lower alkanoyl, aryl, aralkyl or substituted aryl in which the substituent is halo, lower alkyl or lower alkoxy;

$R_4$ is $OR_2$ or $N(R_2)_2$;

$n = 0$ or 1;

$n' = 1$ to 7; and

$n'' = 0$, 1 or 2.

2. A compound of the formula:

$$Ar_1 - X - Ar - Z - (R)_{n'}$$

and salts thereof,

wherein

$Ar_1$ is quinolyl;

Ar is a phenyl, pyridyl or quinolyl ring;

$X = -O(CHR_1)_n-$, $-\underset{(O)_{n''}}{\underset{\|}{S}}(CHR_1)_n-$, $NR_2(CHR_1)_n-$, alkylene of up to 2 carbon atoms in the principal chain and up to a total of 4 carbon atoms,

$$-C(R_1)=C(R_1)-,\ -C\equiv C-, -\underset{O}{\underset{\|}{C}}(CHR_1)_n-,$$

$$-\underset{OH}{\underset{|}{C}}H(CHR_1)_n-,\ -CH=N-,\ -\underset{O}{\underset{\|}{C}}-O-,\ -\underset{O}{\underset{\|}{C}}-S-, -\underset{O}{\underset{\|}{C}}-N(R_1)-;$$

# ANTI-INFLAMMATORY/ANTI-ALLERGIC COMPOUNDS

This invention relates to new chemical compounds possessing valuable pharmaceutical activity. More particularly, the invention relates to novel lipoxygenase inhibitor compounds possessing anti-inflammatory and anti-allergic activities.

The present new compounds are of the formula:

$$Ar_1 - X - Ar - Z - (R)_{n'} \qquad \text{I}$$

and salts thereof,

wherein

$Ar_1$ is a nitrogen, sulfur or oxygen heterocyclic ring or an aromatic hydrocarbon ring such as phenyl, naphthyl, phenanthryl or anthryl;

Ar is a phenyl ring or a nitrogen, oxygen or sulfur heterocyclic ring;

$X = -O(CHR_1)_n-$, $-\underset{(O)_{n''}}{\overset{}{S}}(CHR_1)_n-$, $-NR_2(CHR_1)_n-$ alkylene of up to 2 carbon atoms in the principal chain and up to a total of 4 carbon atoms, $-C(R_1)=C(R_1)-$, $-C{\equiv}C-$, $-\underset{O}{\overset{}{C}}(CHR_1)_n-$, $-\underset{OH}{CH}(CHR_1)_n-$, $-CH=N-$, $-\underset{O}{C}-O-$, $-\underset{O}{C}-S-$, or $-\underset{O}{C}-N(R_1)-$;

Z is an alkylene chain containing up to 10 carbon atoms in the principal chain and a total of up to 12 carbon atoms and from 0 to 2 double bonds and the said alkylene chain may be attached to Ar through an oxygen, sulfur or amino nitrogen atom, and when $n' \geq 2$, one of the R substituents may be halogen on an omega carbon of the alkylene chain Z;

when $n'=1$, R is a substituent attached to one of the carbon atoms of Z selected from the group consisting of =O, $OR_3$, $SR_3$, $N(R_2)_2$, $R_1$ and $-COR_4$, and when $n' \geq 2$ and R is as previously defined and the additional R is a substituent attached to one of the carbon atoms of Z selected from the group consisting of =O, $OR_3$, $SR_3$, $N(R_2)_2$, $-COR_4$ and halo;

$R_1$ is H or $CH_3$;

$R_2$ is H, lower alkyl, aryl or aralkyl;

$R_3$ is H, lower alkyl, lower alkanoyl, aryl, aralkyl or substituted aryl in which the substituent is halo, lower alkyl or lower alkoxy;

$R_4$ is $OR_2$ or $N(R_2)_2$;

$n = 0$ or 1;

$n' = 1$ to 7; and

$n'' = 0$, 1 or 2.

In the foregoing description, the lower alkyl and alkanoyl groups contain up to 6 carbon atoms which may be in the normal or branched configuration, including methyl, ethyl, propyl, isopropyl, butyl, isobutyl, amyl, isoamyl, hexyl, and the like. The aryl group is preferably phenyl, and aralkyl is benzyl. The alkanoyl group is preferably acetyl.

As employed herein, the expression "nitrogen, sulfur or oxygen heterocyclic ring" is meant to include those heterocyclic rings which include at least one sulfur, nitrogen or oxygen atom, but which may include one or several of the said atoms. This expression also is intended to include the so-called "benzo" heterocyclics. Representative heterocyclic rings include furan, thiophene, pyrrole, pyridine, pyrazine, thiazole, oxazole, quinoline, indole, benzothiophene, benzofuran, benzoxazole and the like, as well as N-oxides of the nitrogen heterocyclics.

Ar is preferably a monocyclic ring, e.g., phenyl, pyridine, thiophene, furan and pyrrole, while $Ar_1$ in one embodiment is preferably a bicyclic nitrogen, oxygen or sulfur heterocyclic ring, e.g., quinoline, indole, benzofuran and benzothiophene and in another embodiment is preferably a monocyclic or bicyclic non-heterocyclic ring, e.g. phenyl or naphthyl. These rings are attached in their respective positions in the molecule of the present new compounds through any available carbon of the ring, but preferably through the 2-position of $Ar_1$.

Ar and $Ar_1$ may be fully substituted or less than fully substituted, e.g. mono- or di- or tri- or tetra-substituted with a variety of substituents such as H, $CH_3$ lower alkyl, aryl, aralkyl, halo, hydroxy, lower alkoxy, $CF_3$, carboxy, alkylcarboxy, arylcarboxy, alkylcarbalkoxy, alkanoyl, formyl, oxo, nitrilo, amino, aminoalkyl, alkylamine, carboxamide, aryloxy, nitro, sulfonyl, sulfonamide, thio or alkylthio. It is preferred that the said substituents be present in intermediate compounds used in forming the final products as described in the disclosure of methods of preparation which follows. The substituents may be present on any of the available positions of the ring

systems representative of Ar and $Ar_1$. Of course, substituents which are reactive under the synthetic conditions employed should be blocked using appropriate blocking groups which are readily removable after formation of the desired products. Such blocking groups are well known to those skilled in this art.

The alkylene chains represented by Z can be normal or branched chains in which the branches are preferably methyl or ethyl and include those in which two such groups, e.g., methyl, are on the same carbon atom. The alkylene chains preferably contain up to 8 carbon atoms whether branched or normal. The alkylene chain may contain up to 2 double bonds and may be attached directly to the Ar ring through an oxygen, sulfur or amino nitrogen atom. In addition to substituent R, which is attached to one of the carbon atoms as depicted in the foregoing formula, other substituents such as halogen (F, Cl, Br or I) can be present on the alkylene chain, particularly on the terminal carbon.

Of the various groups representative of X in the foregoing formula, the preferred are those in which n = 1, and especially those which include an oxygen function, particularly the group $-O(CHR_1)_n-$ in which n is 1 and $R_1$ is H. The disposition of the said groups between Ar and $Ar_1$ is not critical, e.g., the $-O(CHR_1)_n-$, can be attached with the oxygen directly on $Ar_1$ or on Ar. Preferably, the oxygen of $-O(CHR_1)$ is attached to $Ar_1$.

-5- 

In the most preferred compounds of this invention, Ar is phenyl, $Ar_1$ is 2-quinolyl, phenyl or naphthyl, X is $-OCH_2-$, Z is alkylene of from about 5-8 carbon atoms and R is an oxygen-containing group, preferably, hydroxy, and n' is 1.

The present new compounds can be prepared by art-recognized procedures. For compounds in which $X = -O(CHR_1)_n$, any of the standard ether forming reactions can be employed as illustrated by the following general procedures:

a) $Ar_1(CHR_1)_nY$ + HO-AR-Z-$(R)_{n'}$

b) $Ar_1(CHR_1)_nOH$ + Y-Ar-Z-$(R)_{n'}$

c) $Ar_1Y$ + $HO(CHR_1)_n$-Ar-Z-$(R)_{n'}$

Formula I - wherein $X=-(CHR_1)_nO-$

In these modifications, Y is a leaving group, most commonly halogen, preferably Cl, Br or I, which forms HY with the hydrogen of the alcohol. Illustrative of processes a) and b), 2-chloromethylquinoline or chloromethylbenzene is condensed with 1-(3-hydroxyphenyl)-1-pentanol and 2-hydroxymethyl-quinoline or hydroxymethylbenzene is condensed with 1-(3-bromophenyl)-1-pentanol. Illustrative of process c) is the condensation of 2-bromopyridine or bromobenzene with 5-(1-hydroxyhexyl) furfuryl alcohol to form the corresponding pyridyl-furylmethyl ether or phenyl-furylmethyl ether. Alternatively,

the leaving group can be a sulfonate ester group, such as the tolyl sulfonate group, which forms toluenesulfonic acid with the hydrogen of the OH group. In all cases where an acid is the by-product, it is preferred to use an acid acceptor to neutralize the acid in the reaction mixture. Such acid acceptors are well-known in the art and include various alkali and alkaline earth metal carbonates and bicarbonates, e.g., sodium carbonate, potassium bicarbonate, cesium carbonate and like, alkali metal alkoxides such as sodium ethoxide, potassium ethoxide and the like, as well as organic amines such as pyridine, pyrrole, dimethylamine, and similar such amines. Of course, when $Ar_1$ and/or Ar includes a basic nitrogen heteroatom, this can serve as the acid acceptor and the product is obtained as an acid salt.

To form compounds in which X is $-\underset{(O)_{n''}}{\overset{}{S}}(CHR_1)_n$ the same reactions can be used employing the corresponding mercaptans in lieu of the alcohols. The sulfoxides and sulfones (where n" = 1 or 2) can be formed by the known reaction with peroxides such as hydrogen peroxide or benzoylperoxides.

For compounds in which X is $-NR_2-(CHR_1)_n-$, the corresponding amino starting compounds are employed to form the desired amino compound. Thus, 2-aminopyridine is condensed with 3-(1-hydroxyhexyl)benzylbromide to form 2-[3-(1-hydroxyhexyl)benzylamino]pyridine. Alternatively, 2-quinolylmethyl bromide is condensed with 3-(1-hydroxyhexyl)aniline to form 2-[3-(1-hydroxyhexyl) anilinomethyl]quinoline. Aniline is condensed with 3-(1-hydroxyhexyl)benzylbromide to form 2-[3-(1-hydroxyhexyl)benzylamino]benzene and benzyl bromide is condensed with 3-(1-hydroxyhexyl)aniline to form 2-[3-(1-hydroxyhexyl) anilinomethyl]benzene.

0181568

-7-

Compounds in which X is -CH=N- are so-called anils and are prepared by condensing an aldehyde with the corresponding amine. Thus, 2-aminopyridine can be condensed with 5-(1-hydroxybutyl)furfural to form the corresponding anil. Alternatively, thiophene-2-aldehyde can be condensed with 5-(1-hydroxypentyl) pyrrolylmethylamine to form the corresponding anil. Aniline can be condensed with 5-(1-hydroxybutyl)furfural to form the corresponding anil and thiophene-2-aldehyde can be condensed with 5-(1-hydroxypentyl) benzylamine to form the corresponding anil.

For those compounds in which X is $-\underset{O}{\underset{\|}{C}}-O-$, $-\underset{O}{\underset{\|}{C}}-S-$ or $-\underset{O}{\underset{\|}{C}}NR_1-$, the acid $Ar_1COOH$ is esterified with $HO-Ar-Z(R)_{n'}$, or the corresponding mercaptans, and the amide is formed by reaction of a suitable derivative of the acid with the amine, $HNR_1Ar-Z(R)_{n'}$. Since the group $-Z(R)_{n'}$ may contain a group reactive with the aforesaid acid derivatives, it is preferred that the desired group $-Z(R)_{n'}$ be formed after the acylation reaction is completed. This can be accomplished by providing a group convertible to the desired group in the Ar nucleus. Such convertible groups include, for example, the nitrilo and formyl groups, which after acylation, can be converted to aminoalkyl in

-8-

the case of the nitrilo group or to hydroxyalkyl in the case of the formyl group, e.g., by reaction with a Grignard reagent to form a secondary alcohol of any desired carbon length. The position of the amino or alcohol hydroxy groups can be set by merely selecting the length of carbon chain (where present) separating the nitrilo or formyl group from the Ar ring carbon as should be obvious to those skilled in the art.

Of course, the group $-Z(R)_{n'}$ may contain groups which are non-reactive in formation of the X linkage between Ar and $Ar_1$ and such groups as are non-reactive may be present on the Ar nucleus before such formation, e.g., ether groups such as alkoxy, alkymercapto, phenoxy, and the like, keto-carbonyl, alkoxy-carbonyl and carboxamido groups. After formation of the X-linkage, such non-reactive groups can be converted to 1°, 2° or 3° alcohol groups by reduction of a carbonyl or reaction with a Grignard reagent with the carbonyl or by reduction of an ester group, $COOR_4$.

For compounds in which Z is ethylenic, $Ar_1CH_2Y$ is condensed with $R_1\underset{\underset{O}{\|}}{C} - ArZ(R)_{n'}$ to form the desired ethylenic compound. From those in which $R_1$ is H, the corresponding acetylenic compounds can be obtained by halogenation to form the dihalo compound and dehydrohalogenation to remove 2 equivalents of hydrogen halide.

Thus, the compounds of the present invention can be prepared by various synthetic methods as exemplified by the following:

- 9 -

1. Condensation of a compound of formula II

$$Ar_1-X' \qquad II$$

in which X' is $-CH_2Hal$, Hal, OH, $CH_2OH$, SH, or $CH_2SH$; with a compound of formula III

$$X''-Ar-Z-(R)n' \qquad III$$

in which X" is $-CH_2Hal$, Hal, OH, $CH_2OH$, SH or $CH_2SH$, through elimination of X' and X" with a residue Z remaining after condensation. Such condensation reactions are known and occur between, for example, $-CH_2Hal$ and -OH with the elimination of hydrogen halide and formation of the bridging group $-CH_2O-$ between the two rings of the resulting product. Substituting simply halogen in place of $-CH_2Hal-$ results in the diphenyl ether structure (where X is -O-). This reaction is known an Ullman ether synthesis. Substituting the analogous sulfuric intermediates provides the sulfur analogs of the aforesaid compounds (where X is -S- or $-CH_2S-$).

A variation of this synthetic route employs the Grignard reagent in place of the halogen-containing substituent.

2. When X is unsaturated hydrocarbyl $[-C(R_1)=C(R_1)-$ or $-C{\equiv}C-$, a compound of formula II in which X' is vinyl or acetylenyl is condensed with a compound of formula III in which X" is halogen, usually in the presence of an active metal (Na, Li, etc.). Alternatively, X' and X" of course can be reversed, i.e., X' can be halogen and X" the unsaturated hydrocarbyl group.

3. When Z is -CH=N-, X' and X" can be -CHO and $NH_2$ and anil formation will result in production of the final product.

4. When Z is an amide or ester linkage, i.e., -COZ-, X' and X", of course, are COOH, and OH or NH on either formula II or formula III, by classical ester or amide formation methods.

5. When Z is a carbonyl, i.e., C=O, X' and X" are H and an acylating group on either formula II or formula III by classical ketone formation reaction, e.g., by Friedl-Crafts reaction; or alternatively by oxidation of the compound in which Z is -CHOH-.

6. When Z includes $-NR_4-$ by classical methods of forming diphenyl amines which include reaction between the compound (either of formula II or III) in which X' or X" is $-NR_4H-$ with the compound (formula II or III) in which X' or X" is halogen.

7. Alternatively, substituent -Z-(R)n' can be added to compounds of formula IV

Ar,-X-Ar  IV

using classical acylation or alkylation condensation reactions, for example, an acylating derivative of an acid

HOO-Z

with a Friedl-Crafts catalyst to provide compounds in which

R is = O

from which compounds in which R is other than O can be prepared by known reactions, e.g., reduction of the carbonyl oxygen to -OH using lithium aluminum hydride and similar reducing agents.

Substituents on $Ar_1$ can be present in the starting intermediates in the foregoing synthetic routes or alternatively can be added by suitable substitution reactions using the starting compounds of formula I herein in which $Ar_1$ is substituted with hydrogen.

The described reactions are performed using classical organic reactions known to those skilled in the art. Of course, reactive groups where present are preferably blocked by known methods to avoid competing or secondary reactions.

The aforesaid reactions are carried out in an organic solvent for the reactive starting materials at temperatures ranging from room temperature up to the reflux temperature of the reaction mixture. In condensation reactions in which hydrogen halide is formed, a hydrogen halide acceptor, i.e., usually a basic compound such as an organic amine, is present to facilitate the reaction. In those reactions where a Friedl-Crafts catalyst is employed, aluminum chloride or zinc-chloride are generally useful.

The final products are recovered from the reaction mixtures and subsequently purified by art-recognized procedures, e.g., column chromatographic techniques.

The compounds of the invention containing basic nitrogen form salts with acids, both organic and inorganic acids. Of particular value are salts with pharmaceutically-acceptable acids especially in dosage forms predicated on aqueous systems where the enhanced water solubility of the salts is most advantageous. Salts formed with pharmceutically unacceptable acids are also useful in the isolation and purification of the basic nitrogen-containing present new compounds. Salts include those formed with hydrochloric, sulfuric, nitric, perchloric, benzenesulfonic, toluenesulfonic, phosphoric, acetic, malic, malonic, tartaric and similar such acids.

The compounds of the invention also exist in stereoisomeric forms due to the presence of asymmetric centers in the molecule, especially in the group $-Z(R)_n$, and in the linkage -X- (where $R_1$ is other than H) or in other parts of the molecule. This invention contemplates the stereoisomers individually or in mixtures or as the racemic compound. The individual stereoisomers can be obtained by standard resolution procedures known to those skilled in the art or by stereospecific synthesis.

0181568



The compounds of the present invention can be administered to the host in a variety of forms adapted to the chosen route of administration, i.e., orally, or parenterally. Parenteral administration in this respect includes administration by the following routes: intravenous, intramuscular, subcutaneous, intraocular, intrasynovial, transepithelially including transdermal, opthalmic, sublingual and buccal; topically including opthalmic, dermal, ocular, rectal and nasal inhalation via insufflation and aerosol and rectal systemic.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring

agent such as peppermint, oil of wintergreen, or cherry flavoring. When the doage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It may be stable under the conditions of manufacture and storage and must be preserved against the contaminating action of microorganisms such as bacteria and

fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

The therapeutic compounds of this invention may be administered to a mammal alone or in combination with pharmaceutically acceptable carriers, as noted above, the proportion of which is determined by the solubility and chemical nature of the compound, chosen route of administration and standard pharmaceutical practice.

The physician will determine the dosage of the present thereapeutic agents which will be most suitable for prophylaxis or treatment and it will vary with the form of administration and the particular compound chosen, and also, it will vary with the particular patient under treatment. He will generally wish to initiate treatment with small dosages by small increments until the optimum effect under the circumstances is reached. The therapeutic dosage will generally be from 0.1 to 100 μM/day or from about 0.1 mg to about 50 mg/kg of body weight per day and higher although it may be administered in several different dosage units. Higher dosages are required for oral administration.

The following examples are illustrative.

- 17 -

## EXAMPLE 1

2-[3-(1-Hydroxy-2-methylhexyl)phenoxymethyl]quinoline

A. A solution of the ketone, 2-(3-hexanoylphenoxymethyl)-quinoline (prepared from 3-hexanoylphenol and 2-chloromethyl quinoline) (6 g, 0.018 mol) in dry THF (25 ml) was added slowly to a mildly refluxing suspension of sodium hydride (1.3 g, 0.054 mol) in dry THF (75 ml) containing excess methyl iodide (10.3 g, 0.073 mol). After the addition of the ketone was complete (30 min.), the reaction mixture was refluxed for 4 hours. After cooling the mixture to room temperature, excess NaH was destroyed by adding methanol. All volatiles were removed at the rotary evaporator and the residue was taken up in ethyl acetate. The organic extract was washed with water and brine, and dried over anhydrous magnesium sulfate. Upon removal of all solvent, the mono-methylated ketone (2-[3-(2-methylhexanoyl) phenoxymethyl]quinoline, was obtained as a yellow oil weighing 8 g.

B. This ketone (8 g) was next reduced with sodium borohydride (1.4 g, 0.036 mol) in ethanol (100 ml) at room temperature for about 15 hours. A little water and methanol was added to the reaction mixture, and then most of the volatiles were removed at the rotary evaporator. The residue was thoroughly extracted with ethyl acetate, and the organics were washed with water, brine, and then dried over anhydrous $MgSO_4$. All solvent was removed to leave a yellow oil (6 g) which was chromatographed on silica gel using 20% ethyl acetate in hexane as eluent. The desired mono methylated alcohol was obtained as a yellowish solid (1.5 g), mp. 64-67°C.

## EXAMPLE 2

2-[3-(1-Hydroxy-2,2-dimethylhexyl)phenoxymethyl]quinoline

A. The ketone 2-(3-(2,2-dimethylhexanoyl)phenoxymethyl)quinoline (4 g) was treated with sodium borohydride (0.9 g) in ethanol (70 ml) at room temperature for about 15 hours. The solution was cooled in an ice bath, and excess of borohydride was destroyed by adding cold, dil HCl solution dropwise. After all the reducing agent was destroyed, the pH of the solution was adjusted to about 5-6, and then most of the volatiles were removed. The residue was taken up in ethyl acetate, and the organics were washed with water, brine and dried over $MgSO_4$. The crude alcohol was isolated by removing all solvents. The pure alcohol (1.6 g, 114-116°C.,), was isolated by chromatography on silica gel using 20% ethyl acetate in hexane as eluent.

B. Preparation of 2-(3-(2,2-dimethylhexanoyl)phenoxymethyl quinoline (starting compound for A. procedure):

A mixture of 2-chloromethylquinoline (12.1 g), 3-(2,2-dimethylhexanoyl)phenol (15 g), finely powdered anhydrous potassium carbonate (20 g) and potassium iodide (0.05 g) in dry acetone (300 ml) was refluxed overnight. The suspension was filtered, and most of the volatiles were removed at the rotary evaporator. The residue was taken up in ethyl acetate, washed with 5% NaOH solution,water, brine,and the extract was dried. On removal of all solvent, the crude ether was obtained as a yellow oil. This oil was purified on HPLC (Prep. 500, Waters) using 10% ethyl acetate in hexane as eluent. The pure material was isolated in about 48% yield (12 g) as a light yellow oil.

C. Synthesis of 3-(2,2-dimethyl hexanoyl)phenol

The benzyl ether of the desired phenol (26 g) was taken in methanol (150 ml) and this solution was hydrogenated in a Parr apparatus under an atmosphere of hydrogen (45-50 lbs./sq.in.) in the presence of 10% palladium on charcoal (10 g). After 20-24 hours, the suspension was filtered on celite, all methanol was removed to obtain the desired phenol in about 83% yield (15.4 g) as a coloress liquid.

The benzyl ether starting compound was prepared by oxidation of 3-(1-hydroxyhexyl)phenyl benzyl ether to the corresponding ketone followed by alkylation with methyl iodide to form the 2,2-dimethyl compound.

In similar manner, using appropriate starting materials and reagents, the following compounds and the corresponding ketones are prepared:

2-[3-(1-hydroxy-2-isobutylhexyl)phenoxymethyl]benzofuran;
2-[3-(1-hydroxy-2,2-diethylpentyl)phenoxymethyl]thiophene; and
2-[3-(1-hydroxy-2-methylhexyl)phenoxymethyl]benzothiophene.

EXAMPLE 3

2-[[3-(1-Hydroxyhexyl)phenoxy]methyl]pyridine hydrochloride

A suspension of 3.3 g of picolyl chloride hydrochloride (Aldrich 16,270-1), 1-(3-hydroxyphenyl)-1-hexanol (3.9 g), cesium carbonate (16.3 g), cesium iodide (trace) and acetone were refluxed for 40 hours. The reaction was filtered through a pad of celite and silica gel and the solvent removed *in vacuo*. The remaining oil was dissolved in ethyl ether, filtered through celite and silica gel and treated with ethereal hydrochloric acid. The resulting white precipitate was filtered, washed (ethyl ether) and dried giving 4.2 g (66% yield) of solid, m.p. 164-165°C.

In like manner as above using appropriate starting materials and reagents, the following compounds were prepared:

3-[[3-(1-Hydroxyhexyl)phenoxy]methyl]pyridine hydrochloride, m.p. 162-163°C;

2-[[3-(1-Hydroxyhexyl)phenoxy]methyl]quinoline hydrochloride, m.p. 90-95°C; and

4-[[3-(1-Hydroxyhexyl)phenoxy]methyl]pyridine hydrochloride, m.p. 160-160.5°C.

EXAMPLE 4

Methyl 6-[[3-(1-hydroxyhexyl)phenoxy]methyl]picolinate

A solution of 6-[[3-(1-hydroxyhexyl)phenoxy]-methyl]picolinyl nitrile (1.1 g), methanol (50 ml) and cesium carbonate was stirred at room temperature overnight. The reaction mixture was diluted with 0.1N hydrochloric acid. After stirring for three hours, the methanol was removed *in vacuo* and the remaining suspension was extracted with chloroform. The organic extract was dried ($MgSO_4$) and concentrated to an oil (7.6 g, 94% yield).

In like manner as above, using appropriate starting materials and reagents, the following compounds were prepared:

Ethyl 2-(3-(2-quinolinylmethoxy)phenoxy)propionic acid;
Ethyl 2-(3-(2-quinolinylmethoxy)phenyl)propionic acid.

## EXAMPLE 5

2-(3-(2-(2-Hydroxy)heptyl)phenoxymethyl)quinoline hydrochloride

A mixture of 2-chloromethyl quinoline hydrochloride (3.1 g) and 2-(3-hydroxyphenyl)-2-heptanol (3 g) in acetone (300 ml) containing potassium carbonate (20 g) and potassium iodide (0.2 g) was refluxed overnight. The reaction mixture was cooled, and most of the volatiles were removed at the rotary evaporator. The residue was taken up in ethyl acetate, and was washed with sodium hydroxide solution (5%) and brine. The organic layer was dried over magnesium sulfate and all solvent was removed to leave the crude product (weighing about 6 g), which was purified by chromatography (silica gel; 20% ethyl acetate in hexanes). The free base, isolated as an oil, was dissolved in dry ether, and treated with hydrogen chloride gas to give the pure desired salt as a white solid, m.p. 125°C. (dec.).

In like manner as described above, using appropriate starting materials and reagents, 2-(3-(2-(2-hydroxy)heptyl)phenoxymethyl)pyridine hydrochloride, m.p. 160-165°C., was prepared.

0181568

-23-

EXAMPLE 6

2-[3-(1-Hexenyl)phenoxymethyl]quinoline

Sodium ethoxide was prepared by dissolving sodium metal (0.9 g) in absolute ethanol (50 ml). 3-Hydroxy-benzaldehyde (5 g) in alcohol (50 ml) was added to sodium ethoxide and the mixture was refluxed for one hour. The reaction mixture was cooled, and a solution of 2-chloromethyl quinoline (7.3 g) in alcohol (50 ml) was added. The mixture was refluxed for one day. All volatiles were removed, and the residue was taken up in ethyl acetate. The organic extract was washed with aqueous sodium hydroxide (5%) solution, water and brine. After drying, all solvent was removed. The residual oil was chromatographed on silica gel using 15% ethyl acetate in hexane as eluent and the pure aldehyde was isolated.

N-pentyl-triphenylphosphonium bromide (3.1 g), prepared by refluxing a toluene solution of 1-bromopentane and triphenylphosphine, and filtering the white phosphonium salt, was dissolved in tetrahydrofuran (100 ml.) The solution was cooled to 0°C., and n-butyl lithium (7.6 mmol; 2.3 M solution in hexane) was added. The mixture was stirred at room temperature for one hour, then cooled again to 0°C., and a solution of the above aldehyde (2 g) in tetrahydrofuran (35 ml) was added. The reaction mixture was stirred at room temperature overnight. Most tetrahydrofuran was removed, and the residue was taken up in ethyl acetate. The organic layer was washed with water, brine, and dried over magnesium sulfate. All solvents were removed, and the crude product was purified on silica gel using 15% ethyl acetate in hexane as eluent. The desired compound was isolated as a light yellow liquid (0.8 g).

-24-

EXAMPLE 7

2-[3-(1-Hydroxyethyl)phenoxymethyl]quinoline

A mixture of 3-(1-hydroxyethyl)phenol (7 g), 2-chloromethylquinoline (9 g), potassium carbonate (7 g) and potassium iodide (0.3 g) in acetone (75 ml) was refluxed overnight. The reaction mixture was poured into water, and then thoroughly extracted with ethyl acetate (3 x 30 ml). The organic extract was washed with NaOH solution (1 N), water, brine, and then dried over $MgSO_4$. All volatiles were removed to obtain a dark brown liquid, which was chromatographed on silica gel (12% ethyl acetate in hexanes) to leave the desired product as a light yellow liquid.

EXAMPLE 8

2-[3-(1-N-methylaminohexyl)phenoxymethyl]quinoline

A solution of 2-[3-(hexanoyl)phenoxymethyl]-quinoline (2.8 g), 40% aqueous methylamine (1.5 ml) and methanol adjusted to pH 6 with 5% aqueous hydrochloric acid is treated with a methanolic solution of sodium cyanoborohydride. The reaction is stirred overnight. The methanol is removed *in vacuo* and the remaining mixture is extracted with methylene chloride. The organic extract is dried ($MgSO_4$) and concentrated to an oil.

In like manner as above, using appropriate starting materials, the following compounds can be prepared:

2-[3-(1-*n*-butylaminohexyl)phenoxymethyl]quinoline; and
2-[3-(1-N,N-dimethylaminohexyl)phenoxymethyl]pyridine.

EXAMPLE 9

2-[3-(2-hydroxy-2-heptyl)phenoxymethyl]quinoline

To an ethereal solution of 2-(3-hexanoylphenoxymethyl)quinoline (3.2 g) (prepared by oxidation of the corresponding alcohol with pyridinium chlorochromate in CH Cl ) is added slowly an excess of a 3M solution of methylmagnesium bromide (2.5 g; 7.0 ml) in ether and the mixture stirred overnight. A saturated solution of ammonium chloride is added dropwise to the well-stirred reaction mixture until the solution became clear, and a gray-white solid coagulated to form a hard cake. The liquid was filtered, and the residue was washed with more ether. The ether layer was separated from the aqueous layer, washed with brine, and dried over magnesium sulfate. All volatiles were removed to give an oil, which was purified by chromatography on silica gel (6% ethyl acetate in hexane) to get the desired tertiary alcohol as a clear, colorless liquid.

In the same manner, using appropriate starting materials and reagents, the following compounds are prepared:

2-[3-(2-hydroxy-2-heptyl)phenoxymethyl]pyridine; and

2-[3-(1-hydroxy-2,2-dimethylbutyl)phenoxymethyl]quinoline.

EXAMPLE 10

3-(3-(1-Hydroxyhexyl)phenoxymethyl)quinoline

To a solution of 3-chloromethylquinoline (10 g, 0.056 mol; prepared from 3-hydroxymethylquinoline and thionyl chloride) and 3-(1-hydroxyhexyl)phenol (12 g, 0.062 mol) in DMSO (200 ml) was added a 2N solution of sodium hydroxide (31 ml, 0.062 mol). The solution became warm (40-45°C.). This was allowed to stir at room temperature for 5 hours. The dark reaction mixture was poured into water, and the aqueous solution was thoroughly extracted (4 x 50 ml) with ethyl acetate. The organic extract was washed with 1N NaOH solution, water and then brine. After drying over anhydrous magnesium sulfate, all volatiles were removed. The dark residual liquid on trituration with ether-petroleum ether (1:2) yielded a beige colored solid, which on recrystallization from hexane-ethyl acetate gave the desried material (12.9 g, 69%) as an off-white solid.

In a similar manner, starting from 6-chloromethylquinoline and 3-(1-hydroxyhexyl)phenol, the other isomeric compound,6-(3-(1-hydroxyhexyl)phenoxymethyl) quinoline, can be prepared.

EXAMPLE 11

2-(3-(2-Quinolinylmethoxy)phenoxy)propionic acid (hydrochloride salt)

Ethyl 2-(3-(2-quinolinylmethoxy)phenoxy)propionate (3.6 g; m.p. 58-60°C.) was taken up in 10% hydrochloric acid solution (180 ml), and the mixture was heated at 100°C. for 26 hours. On cooling the reaction mixture in an ice bath, the desired carboxylic acid crystallized as a hydrochloride salt (3.6 g), which was recrystallized from ethanol to give the desired acid, as its hydrochloride salt, as colorless crystals, (2.2 g; m.p. 184-185°C.).

In a similar manner, starting from ethyl 2-(3-(2-quinolinylmethoxy)phenoxy propionate (2.5 g) and hydrochloric acid (90 ml, 4N), 2-(3-(2-quinolinylmethoxy)-phenoxypropionic acid (hydrochloride salt; 1.3 g; m.p. 136-138°C.) can be prepared.

EXAMPLE 12

2-(3-(1-Hydroxyhexyl)benzyloxymethyl)quinoline

A mixture of 2-hydroxymethylquinoline (8 g, 0 05 mol) and 3-(1-hydroxyhexyl)benzyl p-toluenesulfonate (18.2 g, 0.05 mol; in methylene chloride (200 ml) containing triethylamine (15 ml) was stirred at 0°C. for 4 hours, and then allowed to warm up to room temperature. This mixture was stirred for two more hours at this tmperature, and then poured into water. The organic layer was separated and washed with dilute sodium hydroxide solution, water and brine. After drying the organics, all volatiles were removed to obtain the crude product as a yellowish brown liquid. This was purified by chromatography on silica gel using 15% ethyl acetate in hexanes to get the pure product as a clear, light yellow liquid (9 g, 52% yield).

EXAMPLE 13

3-(2-Quinolinylmethoxy)phenylacetic acid

A mixture of 2-chloromethyl quinoline (2.3g, 13.3 mmol), 3-hydroxyphenyl acetate (2.2g, 13.3 mmol), excess powdered potassium carbonate (10g) and a catalytic amount of potassium iodide (0.1g) in dry acetone (150 ml) was refluxed overnight (18 hr.). The reaction mixture was filtered, and the filtrate was concentrated, taken up in ethyl acetate, and the organic extract was washed with a 5% NaOH solution and brine. After drying over $MgSO_4$, all volatiles were removed from the organic extract, and the crude oil was chromatographed on silica gel (20% ethylacetate in hexanes) to obtain the methyl ester of the desired product (3.0g).

The above ester (3.0g) was dissolved in 4N HCl solution, and this was refluxed for 4 hours. On cooling in an ice bath, the product crystallized out as the hydrochloride salt. This salt was purified further by recrystallization twice from ethanol. The desired product was obtained as the hydrochloride salt, as slightly pink crystals (2.5g), and slowly decomposes with melting above 180°C.

EXAMPLE 14

2-(3-(6-Phenoxy-1,2,2-trimethyl-1-hydroxyhexyl)phenoxymethyl) quinoline

To a solution of the ketone, 2-(3-(6-phenoxy-2,2-dimethyl hexanoyl)phenoxymethyl) quinoline (3.6g) in dry ether (100 ml) at 0°C, was added dropwise, an ethereal solution of methyl magnesiumbromide (7.7 ml, 23.8 mmol, 3 eqv.) under an atmosphere of nitrogen. The clear orange solution was stirred overnight at room temperature. To this solution, a saturated ammonium chloride solution was added when the magnesium salts precipitated. The ether layer was separated, washed with water and brine, and then dried over $MgSO_4$. All volatiles were removed to obtain the crude product (4.2g). This was purified by chromatography on silica gel (25% ethyl acetate in hexanes, 20% ethyl acetate in hexanes and finally 10% ethyl acetate in hexanes) to obtain the pure product as a yellow liquid (0.9g).

## EXAMPLE 15

2-(3-Hexylphenoxy)methyl quinoline

A solution of 99% pure 2-(3-(1-hexenylphenoxy) methyl) quinoline (1.5g) in ethanol (20 ml) containing 10% Pd-C catalyst (0.5g) was exposed to an atmosphere of hydrogen at 10 psig for one hour. The catalyst was filtered through a bed of celite, and all ethanol was removed to obtain the pure desired saturated compound (1.1g) as a yellow liquid.

-33 -

## EXAMPLE 16

### 1-(3-(2-Quinolinylmethoxy)phenyl)-3-methyl-1,3-butane diol

A mixture of 2-chloromethyl quinoline hydrochloride (10g, 47 mmol), 3-hydroxybenzaldehyde (6.3g, 52 mmol), powdered potassium carbonate (14.5g, 105 mmol), catalytic amounts of cesium carbonate (1.5g) and sodium iodide (0.7g) in dry acetone (150 ml) was refluxed for 14 hours. The solid residue was filtered off, and the filtrate was concentrated and dissolved in ethyl acetate. The organic solution was washed successively with 10% NaOH solution, water and brine and then dried over $MgSO_4$. All volatiles were removed to leave a yellowish solid weighing 14.8g. This was recrystallized from hexanes-ethanol mixture to obtain 4-(3-(2-quinolinylmethoxy)phenyl-4-hydroxy-2-butanone as off-white crystals (11.8g), mp 116-9°C.

To a solution of the above hydroxy-ketone (2.5g) in dry THF (150 ml) was added dropwise, an ethereal solution of methyl magnesium bromide (17.1 mmol, 2.2 eqv.). The reaction mixture was stirred overnight, and then a saturated ammonium chloride solution was added. The precipitated magnesium salts were filtered, and the filtrate was concentrated in vacuo. The desired material was isolated after purification by chromatography on silica gel using 40% ethyl acetate in hexanes, as a yellowish crystalline solid, mp 117-124°C (1.2g).

## EXAMPLE 17

2-(3-(6,6,6-Trifluorohexanoyl)phenoxymethyl) quinoline

A mixture of 3-(5,5,5-trifluorohexanoyl)phenol (6.8g, 27 mmol) and 2-chloromethylquinoline (4.4g, 25 mmol) in DMSO (75 ml) containing 2N NaOH solution (14 ml) was stirred at room temperature overnight (13 hrs.). The dark reaction mixture was poured into water (200 ml) and the aqueous solution was extracted with ethyl acetate (4 x 30 ml). The organic extract was washed with 1N NaOH solution, water and brine and then dried over $MgSO_4$. After removal of all volatiles, a dark liquid was obtained which solidified on standing (7.2g). This solid was chromatographed (3% ethanol in toluene on silica gel) to obtain a beige color solid (4.5g; mp 83-4°C), which was chromatographed again (15% acetone in hexanes on silica gel) to get a white solid (3.1g; mp 86-87°C). This pure material was recrystallized from hexanes-acetone (trace) to leave the desired compound as a colorless, crystalline solid (2.4g), mp 88-88.5°C (first crop; second crop mp 87-88°C).

0181568

-35-

EXAMPLE 18

2-(3-(5,5,5-Trifluoropentanoyl)phenoxymethyl) quinoline

This material was synthesized by refluxing a mixture of 2-chloromethyl quinoline (2.6g, 14.5 mmol), 3-(5,5,5-trifluoropentanoyl)phenol (3.7g, 15.9 mmol), powdered potassium carbonate (6.0g, 43.5 mmol) and a catalytic amount of potassium iodide (0.1g) in dry acetone (50 ml) for 14 hours. After this period, the mixture was filtered, and the filtrate was concentrated. The residue was dissolved in ether, and the ethereal solution was washed with brine, dried over $MgSO_4$, and then concentrated in vacuo. The pure desired ketone was isolated by chromatography on silica gel using 20% ethyl acetate in hexanes as eluent. The product was obtained in 62% yield (3.4g), had a melting point 80-82°C and was a yellowish solid.

EXAMPLE 19

2-(3-(6-Benzyloxy-1-hydroxyhexyl)phenoxymethyl) quinoline

2-Chloromethyl quinoline (3.6g) was refluxed with 3-(6-benzyloxy-1-hydroxyhexyl)phenol (4.2g), powdered potassium carbonate (8g) and potassium iodide (0.2g) in dry acetone (150 ml) overnight (16 hrs.). The suspension was filtered, the filtrat was concentrated in vacuo and then taken up in ethyl acetate. This solution was washed with 5% NaOH solution, water and brine and then dried. All solvent was removed to obtain the crude product which was purified by chromatography (silica gel; 30% ethyl acetate in hexanes). The pure compound was dissolved in ether, and was precipitated as the hydrochloride salt with dry ethereal solution of HCl. The pure salt was filtered, washed with cold water, and dried in vacuo to yield a tan powder (1.5g), mp 86-89°C.

- 37 -



EXAMPLE 20

Methyl 3-(3-(2-quinolinylmethoxy)benzoyl)propanoate

A mixture of pure methyl 4-(3-(2-quinolinylmethoxy) phenyl-4-hydroxy butyrate (1.1g) and activated manganese dioxide (6.2g) in methylene chloride (25 ml) was stirred at room temperature. After 20 hours, no starting material was detected by thin layer chromatography. The manganese dioxide was filtered through celite, and the methylene chloride solution was concentrated in vacuo. The residue was purified by chromatography on silica gel using 15% ethyl acetate in hexanes containing 1% acetic acid as eluent. The desired keto-ester was obtained (0.22g) as an off-white solid, mp 80-81°C.

EXAMPLE 21

Synthesis of 5-phenoxypentyl bromide

To a well-stirred suspension of sodium hydride (15.3g, 0.32 mol; 50% oil dispersion) in dry DMF (500 ml) containing (25g, 0.26 mol), was added dropwise 1,5-dibromopentane (122.2g, 0.53 mol). After the addition was complete, the reaction mixture was stirred at 60-70°C for 4 hours. Most of the DMF was next removed under reduced pressure, and the residue was dissolved in ethyl acetate (250 ml). The organic extract was washed successively with a 5% sodium hydroxide solution, water and brine. After drying the organics over anhydrous magnesium sulfate, all volatiles were removed. The residual yellow liquid was then distilled under reduced pressure, and the pure product was collected at 118-125°C/1 mm as a clear colorless liquid (26g, 40% yield).

Synthesis of 3-(6-phenoxy-1-hydroxyhexyl)phenol

To gently refluxing dry tetrahydrofuran (70 ml) containing magnesium turnings (1.1g, 44 mmol) and a small crystal of iodine, was added dropwise from a dropping funnel a solution of 5-phenoxypentyl bromide (11.0g, 45 mmol) in dry THF (50 ml). After about 5-7 minutes, the Grignard reaction started as indicated by the disappearance of the brown iodine color. The heating was adjusted so that the solution refluxed gently. After the addition of the halide was complete (ca. 30 min.), the clear solution was refluxed for about 1 hour. This solution of the Grignard reagent was cooled to room temperature.

A solution of 3-hydroxybenzaldehyde (5.0, 41 mmol) in dry THF (50 ml) was added dropwise to a well-stirred suspension of sodium hydride (2.5g, 52 mmol; 50% oil dispersion) in dry THF (50 ml) at room temperature. The

temperature was not allowed to go above ca. 40°C. After the addition of 3-hydroxybenzaldehyde was complete (ca. 30 min.) the slightly cloudy solution was warmed to 45-50°C and stirred for 1 hour at this temperature. The resulting clear solution of sodium 3-formyl phenoxide was cooled to room temperature and added dropwise using a cannula, to the well-stirred solution of the Grignard reagent (whose preparation was described above). A thick precipitate immediately formed. This mixture was maintained at 40-45°C during the addition of the sodium salt. After the addition was complete (ca. 2 hours), the reaction mixture was stirred at this temperature for another hour, and then cooled in an ice bath. The excess reactive materials (sodium hydride, Grignard reagent, etc.) were destroyed slowly by carefully adding methanol (30 ml), and then a saturated ammonium chloride solution (350 ml) was added to dissolve all solids. The organic layer was separated, and the aqueous layer was extracted with ether (2 x 75 ml). The combined organics were washed with water, brine, and then dried over anhydrous magnesium sulfate. After drying the extract, all volatiles were removed to obtain a dark liquid. This oil was titurated with a 1:1 mixture of ether and petroleum ether (35-55°C) when an off-white solid was obtained (12g). This can be crystallized from methanol-ethyl acetate to give a white solid, mp 148-9°C.

Synthesis of 2-[3-(6-phenoxy-1-hydroxyhexyl)phenoxymethyl] quinoline

A mixture of 2-chloromethyl quinoline (1.1g; 6.3 mmol), 3-(6-phenoxy-1-hydroxyhexyl)phenol (1.8g, 6.3 mmol) and finely powdered anhydrous potassium carbonate (1.74g, 12.6 mmol) in acetone (40 ml) containing catalytic amounts of finely powdered, anhydrous cesium carbonate

(0.4, 1.3 mmol) and sodium iodide (0.05g, 0.33 mmol) was refluxed overnight (15 hours). The reaction mixture was cooled to the room temperature and filtered. The solid residue was washed thoroughly with acetone (3 x 15 ml), and all the filtrates were combined. All volatiles were removed from the combined acetone filtrate to leave a light yellow viscous liquid which solidifed to an off-white solid on tituration with a 1:1 mixture of ether and petroleum ether (35-55°C). This solid was chromatographed on silica gel with 35% ethylacetate in hexanes to yield the desired product (1.6g, 60%) as a white crystalline solid, mp 83-84.5°C.

EXAMPLE 22

2-[3-(1-Hydroxyhexyl)phenoxymethyl]-1,2,3,4-tetrahydronaphthalane

A mixture of 4.0 g (0.018 mol) 2-(iodomethyl)-1,2,3,4-tetrahydronaphthalene, 3.4 g (0.018 mol) 3-(1-hydroxyhexyl)phenol and 3.4 ml NaOH (5N) in 50 ml DMSO and 20 ml THF was stirred at room temperature for a period of 48 hours. The reaction mixture was concentrated under reduced pressure. The concentrated reaction mixture was poured into water and extracted with ether. The ether extract was washed with water, dried over $MgSO_4$ and concentrated to dryness under reduced pressure. The residue was passed through a silica gel column using hexane/ethyl acetate (3:1) as eluent. Evaporation of eluent gave an oily product (1.3 g).

The product was evaluated for 5-lipoxygenase pathway inhibition (5-LOX/$I_{50}$/$\mu$M) and a value of 6.0 $\mu$M obtained.

0181568

-42-

EXAMPLE 23

2-(3-(1-Hydroxyhexyl)phenoxymethyl)naphthalene

A suspension of 2-bromomethyl naphthalene (5 g, 0.023 mol), 3-(1-hydroxyhexyl)phenol (5 g, 0.026 mol), powdered potassium carbonate (4.5 g, 0.033 mol), sodium iodide (0.4 g, 0.0023 mol) and cesium carbonate (0.7 g, 0.0021 mol) in dry acetone (75 ml) was refluxed overnight (14 hours). The reaction mixture was filtered, the filtrate was concentrated, dissolved in ether, and then washed successively with 1N NaOH solution 6N HCl solution, water and brine. After drying the organics over anhydrous $MgSO_4$, all volatiles were removed. The crude product (5 g) was further purified by chromatography, using 8% ethylacetate in hexanes on silica gel, to yield 3.5 g of the pure desired compound as a colorless liquid.

The compound was evaluated for 5-lipoxygenase pathway inhibition (5-LOX/$I_{50}$ $\mu$M) and a value of 5.0 $\mu$M obtained.

EXAMPLE 24

2-(3-Hexanoylphenoxy)methyl naphthalene

A mixture containing 2-bromomethyl naphthalene (3.0 g, 13.5 mmol), 3-hexanoylphenol (2.6 g, 13.5 mmol), powdered potassium carbonate (19.0 g, 135 mmol) and potassium iodide (0.1 g) in dry acetone (100 ml) was refluxed for two days. Most of the solvent was removed after filtering off the solid residue. The oil was purified by chromatography on silica gel using a 5% ethylacetate in hexane solution as eluent to leave the desired product as a clear, colorless liquid (2.6 g).

When evaluated, the product showed some 5-lipoxygenase inhibition (5-LOX/$I_{50}$/$\mu$M).

The following protocol describes an assay to detect inhibitors of the lipoxygenase pathway. Such inhibitors are believed to be capable of modulating the biosynthesis of the leukotrienes, a property believed to be useful in treating asthma and inflammatory disease states.

Protocol for Detecting Inhibitors of the Lipoxygenase Pathway

A suspension of rat neutrophils in buffer is incubated for 3 minutes at 30°C with [$^{14}C$]-arachidonic acid (AA) and Calcium Ionophore A23187. Citric Acid (2M) is used to quench the reaction. Following the addition of a trace amount of ($^{3}H$)-5-HETE together with an excess of unlabeled 5-HETE to each tube, the mixture is extracted with chloroform/methanol. The organic layer is washed with dilute acid and an aliquot is transferred to glass tubes and dried. The residue is dissolved in a small volume of chloroform and an aliquot is spotted on silica gel TLC sheets, which are developed with an ethyl acetate/isoctane/water/acetic acid solvent system. The 5-HETE spots are visualized with iodine, cut out and placed in scintillation vials for counting. After adjusting for the extraction efficiency, the amount (pmole) of [$^{14}C$]-5-HETE in each of the tubes is quantitated. The net pmoles of 5-HETE are obtained by substracting the pmoles of 5-HETE in the tubes

containing buffer alone (blank) from the pmoles of 5-HETE in the tubes containing buffer and cells (control). The ability of the test compounds to modulate the activity of this enzyme is determined by a decrease or increase in the net amount of 5-HETE produced.

Table I shows the concentration required for 50% inhibition of the 5-lipoxygenase pathway (5-LOX/$I_{50}$µM) for representative compounds according to the present invention.

## TABLE I

$Ar_1 - X - Ar - Z - (R)_{n'}$

| $Ar_1$ | X | Ar | $Z(R)_{n'}$ | 5-LOX $I_{50}$ μM |
|---|---|---|---|---|
| 2-quinolyl | $-CH_2O-$ | $C_6H_4$ * | $3-(CHC_5H_{11})$ **<br>OH | 0.16 |
| 2-quinolyl | $-CH_2O-$ | $C_6H_4$ | $3-(C(CH_3)C_5H_{11})$<br>OH | 0.1 |
| 2-pyridyl | $-CH_2O-$ | $C_6H_4$ | $3-(-CHC_5H_{11})$<br>$NHCH_3$ | 2.2 |
| 2-quinolyl | $-CH_2O-$ | $C_6H_4$ | $3-(-CH=CHC_4H_9)$ ** | 2.4 |
| 2-quinolyl | $-CH_2O-$ | $C_6H_4$ | $3-(CHC_5H_{10}-O-$ 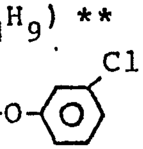 )<br>OH | 0.6 |
| 2-quinolyl | $-CH_2O-$ | $C_6H_4$ | $3-(CH-C_5H_{10}OC_6H_5)$<br>OH | 0.6 |
| 2-quinolyl | $-CH_2O-$ | $C_6H_4$ | $3-(C(CH_3)C_5H_{10}-OC_6H_5)$<br>OH | 0.8 |
| 2-quinolyl | $-CH_2O-$ | $C_6H_4$ | $3-(CHCH(CH_3)C_4H_9)$<br>OH | 0.2 |
| 2-quinolyl | $-CH_2O-$ | $C_6H_4$ | $3-(CHC(CH_3)_2C_4H_9)$<br>OH | 0.27 |
| 2-quinolyl | $-CH_2O-$ | $C_6H_4$ | $3-(C(CH_3)C(CH_3)_2C_4H_9)$<br>OH | 0.27 |

* $C_6H_4$ is phenylene

** $C_4H_9$ and $C_5H_{11}$ are linear alkyl groups

TABLE I - Cont'd.

$Ar_1 - X - Ar - Z - (R)_{n'}$

| $Ar_1$ | X | Ar | $Z(R)_{n'}$ | 5-LOX $I_{50}$ uM |
|---|---|---|---|---|
| 2-quinolyl | -O- | $C_6H_4$ | $4-(\underset{OH}{CH}-C_5H_{11})$ | 0.35 |
| 4-pyridyl | $-CH_2O-$ | $C_6H_4$ | $3-(\underset{OH}{CH}C_5H_{11})$ | 3 |
| 2-pyridyl | $-CH_2O-$ | $C_6H_4$ | $3-(\underset{OH}{CH}C_5H_{11})$ | 0.5 |
| 3-pyridyl | $-CH_2O-$ | $C_6H_4$ | $3-(\underset{OH}{CH}C_5H_{11})$ | 3 |
| 6-(2-carboxy-pyridyl | $-CH_2O-$ | $C_6H_4$ | $3-(\underset{OH}{CH}C_5H_{11})$ | 1.9 |
| 6-(2-cyano-pyridyl | $-CH_2O-$ | $C_6H_4$ | $3-(\underset{OH}{CH}C_5H_{11})$ | 30 |
| 2-quinolyl | $-CH_2O-$ | $C_6H_4$ | $3-(\underset{\overset{\|\|}{O}}{C}C_5H_{11})$ | 0.15 |
| 2-pyridyl | $-CH_2O-$ | $C_6H_4$ | $3-(\underset{OH}{C}(CH_3)C_5H_{11})$ | 0.3 |
| 2-quinolyl | $-CH_2O-$ | $C_6H_4$ | $3-(\underset{OH}{CH}CH_2\underset{\overset{\|\|}{O}}{C}CH_3)$ | 0.18 |
| 2-quinolyl | $-CH_2O-$ | $C_6H_4$ | $4-(\underset{OH}{CH}C_5H_{11})$ | 0.35 |

**TABLE I - Cont'd.**

$Ar_1 - X - Ar - Z - (R)_{n'}$

| $Ar_1$ | X | Ar | $Z(R)_{n'}$ | 5-LOX $I_{50}$, uM |
|---|---|---|---|---|
| 3-carboxyphenyl | $CH_2O$ | $C_6H_4$ | 3-(CHC$_5H_{11}$) with OH on CH | 25 |
| 2-carboxyphenyl | $CH_2O$ | $C_6H_4$ | 3-(CHC$_5H_{11}$) with OH on CH | 30 |
| 2-carboxyphenyl | $CH_2O$ | $C_6H_4$ | 3-(CHC$_4H_9$) with OH on CH | 10 |
| 4-carboxyphenyl | $CH_2O$ | $C_6H_4$ | 3-(CHC$_5H_{11}$) with OH on CH | 21 |
| 3-carbomethoxyphenyl | $CH_2O$ | $C_6H_4$ | 3-(CHC$_5H_{11}$) with OH on CH | 1.2 |
| 2-carbomethoxyphenyl | $CH_2O$ | $C_6H_4$ | 3-(CHC$_5H_{11}$) with OH on CH | 0.6 |
| 2-carbomethoxyphenyl | $CH_2O$ | $C_6H_4$ | 3-(CHC$_4H_9$) with OH on CH | 3.0 |
| 4-carbomethoxyphenyl | $CH_2O$ | $C_6H_4$ | 3-(CHC$_5H_{11}$) with OH on CH | 3.2 |

TABLE I - Cont'd.

$Ar_1 - X - Ar - Z - (R)_{n'}$

| $Ar_1$ | X | Ar | $Z(R)_{n'}$ | 5-LOX $I_{50}$ µM |
|---|---|---|---|---|
| 3-carbomethoxyphenyl | $CH_2O$ | $C_6H_4$ | 2-($CHC_5H_{11}$) OH | 3.2 |
| 3-carboxymethylphenyl | $CH_2O$ | $C_6H_4$ | 3-($CHC_5H_{11}$) OH | |
| 3-carbomethoxymethylphenyl | $CH_2O$ | $C_6H_4$ | 3-($CHC_5H_{11}$) OH | 5.8 |
| 3-carbomethoxyphenyl | $CH_2O$ | $C_6H_4$ | 3-($CHC_5H_{11}$) $OCOCH_3$ | 5.0 |
| 3-carbomethoxyphenyl | $CH_2O$ | $C_6H_4$ | 3-($CHC_5H_{11}$) $OCH_3$ | 10 |
| 3-carbomethoxyphenyl | $CH_2O$ | $C_6H_4$ | 3-($CHC_5H_{11}$) OTHP*** | 23 |
| 2-carbomethoxyphenyl | $CH_2O$ | $C_6H_4$ | 3-($CHC_4H_9$) OTHP | 10 |
| 2-hydroxymethylphenyl | $CH_2O$ | $C_6H_4$ | 3-($CHC_4H_9$) OH | 4 |

***THP is tetrahydro-2H-pyran-2yl-

TABLE I - Cont'd.

$Ar_1 - X - Ar - Z - (R)_{n'}$

| $Ar_1$ | X | Ar | $Z(R)_{n'}$ | 5-LOX $I_{50}$, uM |
|---|---|---|---|---|
| 3-formylphenyl | $CH_2O$ | $C_6H_4$ | $3-(\underset{OH}{CH}C_4H_9)$ | 4.0 |
| 3-cyanophenyl | $CH_2O$ | $C_6H_4$ | $3-(\underset{OH}{CH}C_5H_{11})$ | 17 |
| 3-aminomethylphenyl | $CH_2O$ | $C_6H_4$ | $3-(\underset{OH}{CH}C_5H_{11})$ | |
| 3-carbamylphenyl | $CH_2O$ | $C_6H_4$ | $3-(\underset{OH}{CH}C_5H_{11})$ | 2.0 |
| 4-acetoxymethoxyphenyl | $CH_2O$ | $C_6H_4$ | $3-(CHC_5H_{11})$ | 4.8 |
| phenyl | $CH_2O$ | $C_6H_4$ | $3-(\underset{OH}{CH}C_5H_{11})$ | 2.7 |
| phenyl | $OCH_2$ | $C_6H_4$ | $3-(\underset{OH}{CH}C_5H_{11})$ | 6.7 |
| 3-carbomethoxyphenyl | $CH_2O$ | $C_6H_4$ | $3-(\underset{CH_3}{C}=CHC_4H_9)$ | 5.0 |

TABLE I - Cont'd.

$Ar_1 - X - Ar - Z - (R)_{n'}$

| $Ar_1$ | X | Ar | $Z(R)_{n'}$ | 5-LOX $I_{50}$ uM |
|---|---|---|---|---|
| 3-carbomethoxyphenyl | $CH_2O$ | $C_6H_4$ | $4-(CH=CHC_4H_9)$ | 100 |
| phenyl | $CH_2O$ | $C_6H_4$ | $3-(\underset{O}{\overset{\parallel}{C}}-C_5H_{11})$ | 2.0 |
| phenyl | $CH_2O$ | $C_6H_4$ | $3-[\underset{O}{\overset{\parallel}{C}}C(CH_3)_2C_4H_9]$ | 7.5 |
| phenyl | $CH_2O$ | $C_6H_4$ | $3-[\underset{OH}{CH}C(CH_3)_2C_4H_9]$ | 2.5 |
| phenyl | $CH_2O$ | $C_6H_4$ | $3-[\underset{OH}{CH}CH(CH_3)C_4H_9]$ | 3.0 |
| 3-carbomethoxyphenyl | $CH_2O$ | $C_6H_4$ | $3-[\underset{OH}{CH}C(CH_3)_2C_4H_9]$ | 2.0 |
| phenyl | $CH_2O$ | $C_6H_4$ | $3-[\underset{OH}{C}(CH_3)C_5H_{11}]$ | 1.0 |
| phenyl | $CH_2O$ | $C_6H_4$ | $3-[\underset{OH}{C}(CH_3)C(CH_3)_2C_4H_9]$ | 2.4 |

- 52 -

**TABLE I - Cont'd.**

$Ar_1 - X - Ar - Z - (R)_{n'}$

| $Ar_1$ | X | Ar | $Z(R)_{n'}$ | 5-LOX $I_{50}$ µM |
|---|---|---|---|---|
| phenyl | $CH_2O$ | $C_6H_4$ | 3-($CHC_5H_{11}$) with $NHCH_3$ on C | 22 |
| phenyl | trans CH=CH | $C_6H_4$ | 3-($CHC_5H_{11}$) with OH on C | 4.5 |
| 3-carbamylphenyl | $CH_2O$ | $C_6H_4$ | 3-($CC_5H_{11}$) with =O on C | 0.7 |
| 3-cyanophenyl | $CH_2O$ | $C_6H_4$ | 3-($CC_5H_{11}$) with =O on C | 8.0 |
| phenyl | -O- | $C_6H_4$ | 3-($CHC_5H_{11}$) with OH on C | 1.2 |
| 3-trifluoromethylphenyl | $CH_2O$ | $C_6H_4$ | 3-($CHC_5H_{11}$) with OH on C | 3.0 |

Using the procedures of the foregoing examples, the following compounds are prepared:

2-[3-(1-hydroxyhexyl)phenoxymethyl]quinoline;

2-[3-(1-methyl-1-hydroxyhexyl)phenoxymethyl]quinoline;

2-[3-(1,2-dihydroxyhexyl)phenoxymethyl]quinoline;

2-[3-(2,2-dimethyl-1-hydroxyhexyl)phenoxymethyl]pyridine;

2-[3-(1,2,2-trimethyl-1-hydroxyhexyl)phenoxymethyl]quinoline;

2-[3-(1-methyl-1-hydroxyhexyl)phenoxymethyl]pyridine;

2-[3-(6-[3-chlorophenoxy]-1-hydroxyhexyl)phenoxymethyl]quinoline;

2-[3-(6-phenoxy-1-hydroxyhexyl)phenoxymethyl]quinoline;

2-[3-(6-phenoxy-2,2-dimethyl-1-hydroxyhexyl)phenoxymethyl]-quinoline

2-[3-(6-[3-trifluoromethylphenoxy]-1-hydroxyhexyl)phenoxymethyl]quinoline;

2-[3-(1-hydroxyhexyl)phenoxymethyl]benzofuran;

2-[5-(1-hydroxyhexyl)-2-furyloxy)methyl]pyridine;

4-[5-(1-hydroxyhexyl)-2-thienylmethoxy]quinoline;

2-[6-(1-hydroxyhexyl)-3-pyridyloxy]quinoline;

3-[3-(1-hydroxyhexyl)phenoxymethyl]pyridine;

2-[3-(1-hydroxyethyl)benzylthio]pyridine; and

2-[3-(6-[3-chlorophenoxy]-1-methyl-1-hydroxyhexyl)phenoxymethyl]quinoline;

2-(3-(1-hydroxy-2,2-dimethyl-5,5,5-trifluoropentyl)-phenoxymethyl)quinoline;

2-(3-(1,2,2,-trimethyl-5,5,5-trifluoro-1-hydroxypentyl)phenoxymethyl)quinoline;

2-(3-(2,2-dimethyl-6,6,6-trifluoro-1-hydroxyhexyl)-phenoxymethyl)quinoline;

2-(3-(1,2,2-trimethyl-6,6,6-trifluoro-1-hydroxyhexyl)-phenoxymethyl)quinoline;

2-(3-(2,2-dimethyl-6,6,6-trifluoro-1-hydroxyhexyl)-phenoxymethyl)pyridine;
3-(3-(2,2-dimethyl-6,6,6-trifluoro-1-hydroxyhexyl)-phenoxymethyl)pyridine;
4-(3-(2,2-dimethyl-6,6,6-trifluoro-1-hydroxyhexyl)-phenoxymethyl)quinoline;
2-(3-(6-methoxyhexyl)phenoxymethyl)quinoline;
2-(3-(4-methoxybutyl)phenoxymethyl)quinoline;
2-(4-(4-methoxybutyl)phenoxymethyl)quinoline;
2-(3-(6-phenoxyhexyl)phenoxymethyl)quinoline;
2-(3-(4-methoxybutyl)phenoxy)quinoline;
2-(3-(1,1,1-trimethylacetoxyhexyl)phenoxymethyl)-quinoline;
2-(3-(1-acetoxy-5,5,5-trifluoropentyl)-phenoxymethyl)-quinoline;
2-(3-(1,1,1-trimethylacetoxy-5,5,5-trifluoropentyl)-phenoxymethyl)quinoline;
2-(3-(1,1,1-trimethylacetoxy-6,6,6-trifluorohexyl)-phenoxymethyl)quinoline;
2-(3-(2,2-dimethyl-1,1,1-trimethylacetoxyhexyl)phenoxymethyl)quinoline;
2-(3-(2,2-dimethyl-6,6,6-trifluoro-1,1,1-trimethyl-acetoxyhexyl)phenoxymethyl)quinoline.
3-(3-(2-quinolinylmethoxy)benzoyl)propanoic acid;
5-(3-(2-quinolinylmethoxy)benzoyl)pentanoic acid;
methyl 5-(3-(2-quinolinylmethoxy)benzoyl) pentanoate;
methyl 4-(3-(2-quinolinylmethoxy)phenyl)-4-hydroxy butyrate;
4-(3-(2-quinolinylmethoxy)phenyl)-4-hydroxy butyric acid (and the corresponding lactone);
methyl 6-(3-(2-quinolinylmethoxy)phenyl)-6-hydroxy hexanoate;
6-(3-(2-quinolinylmethoxy)phenyl)-6-hydroxy hexanoic acid (and the corresponding lactone);

5-(3-(2-quinolinylmethoxy)phenyl)-5-hydroxy pentanoic acid (and the corresponding lactone);
methyl 5-(3-(2-quinolinylmethoxy)phenyl)-5-hydroxy pentanoate;
methyl 4-(3-(2-quinolinylmethoxy)benzoyl)butyrate;
4-(3-(2-quinolinylmethoxy)benzoyl)butyric acid;
6-(3-(1-hydroxyhexyl)phenoxymethyl)-1,4-benzodioxan;
2-(3-(1-hydroxyhexyl)phenoxymethyl)-1,4-benzodioxan;
2-(3-(1-hydroxyhexyl)phenoxymethyl)quinoxaline
2-(3-(1-hydroxyhexyl)phenoxymethyl)-3-methyl quinoxaline;
7-(3-(1-hydroxyhexyl)phenoxymethyl)-5-methyl-1,8-naphthyridin-2-ol;
2-(3-(1-hydroxy-6-phenoxyhexyl)phenoxymethyl)quinoxaline;
2-(3-(1-hydroxy-6-phenoxyhexyl)phenoxymethyl)-1,5-naphthyridine;
2-(3-(1-hydroxy-6-phenoxyhexyl)phenoxymethyl)-1,6-naphthyridine;
2-(3-(1-hydroxyhexyl)phenoxymethyl)pyrido(2,3-b)pyrazine;
2-(3-(1-hydroxy-6-phenoxyhexyl)phenoxymethyl)pyrido(3,4-b)pyrazine;
2-(3-(1-hydroxyhexyl)phenoxymethyl)pyrido(3,4-d)pyrimidine;
2-(3-(1-hydroxy-6-phenoxyhexyl)phenoxymethyl)pyrido(4,3-d)pyrimidine;
3-(3-(1-hydroxyhexyl)phenoxymethyl)piperazine-2,5-dione; and
3-(3-(6-phenoxy-1-hydroxyhexyl)phenoxymethyl)-2,5-dimethoxy-3,6-dihydropyrazine.

1-(3-phenoxyphenyl)-1-hexanol;
3-(1-hydroxyhexyl)phenyl-3-trifluoromethylbenzyl ether;
2-(3-(1-hydroxyhexyl)phenoxy methyl naphthalene;
3-(3-hexanoylphenoxy) methyl benzamide;
3-(3-hexanoyl phenxoy) methyl benzonitrile;
benzyl-3-(1-hydroxy-2-methyl hexyl)phenyl ether;
2-[2-(1-hydroxy hexyl)-5-pyridyloxy]benzene;
2-[2-(2-hydroxy hexyl)-3-pyranyloxy]benzene;
2-[2-(2-hydroxy heptyl)-4-thiopyranyloxy] benzene;
2-[2-(2-hydroxy heptyl)-5-quinolyloxy]benzene;
2-(3-(1-hydroxy-6,6,6-trifluorohexyl)phenoxymethyl)-1,4-naphthaquinone;
1-(3-(1-hydroxy-1-methylhexyl)phenoxymethyl)-2-methoxy naphthalene;
1-(3-(6-phenoxy-1-hydroxyhexyl)phenoxymethyl)-4-methoxy naphthalene;
2-(1-(3-(1-hydroxy-2,2-dimethylhexyl)phenoxy)ethyl)naphthalene;
9-(3-(1-hydroxyhexyl)phenoxymethyl)anthracene;
9-(3-(1-hydroxyhexyl)phenoxymethyl)phenanthrene;
Methyl-3-[[3-(1-hydroxyhexyl)phenoxy]methyl]benzoate;
Methyl-2-[[3-(1-hydroxyhexyl)phenoxy]methyl]benzoate;
Methyl-4-[[3-(1-hydroxyhexyl)phenoxy]methyl]benzoate;
Methyl-3-[[2-(1-hydroxyhexyl)phenoxy]methyl]benzoate;
Methyl-3-[[3-(1-hydroxyhexyl)phenoxy]methyl]phenylacetate;
3-[[3-(1-Hydroxyhexyl)phenoxy]methyl]benzonitrile;
Methyl-2-[4-[[3-1-hydroxyhexyl)phenoxy]methyl]phenoxy]acetate;
Benzyl-3-[1-(hydroxy)hexyl]phenyl ether;
Methyl-3-[[3-1-methyl-1-hexenyl)phenoxy]methyl benzoate;
Methyl-3-[[4-(1-hexenyl)phenoxy]methyl benzoate;
Phenyl-3-cyanobenzyl ether;
3-(1-Hydroxyhexyl)phenyl-3-trifluoromethylbenzyl ether;
2-(3-(1-Hydroxyhexyl)phenoxy methyl naphthalene;

2-[[3-(1-hydroxyhexyl]phenoxy]methyl]benzoic acid;

3-[[3-1-hydroxyhexyl)phenoxy]methyl]benzoic acid;

5-[[3-1-hydroxyhexyl)phenoxy]methyl]benzoic acid;

4-[[3-(1-hydroxyhexyl)phenoxy]methyl]benzoic acid;

3-[[3-(1-hydroxyhexyl)phenoxy]methyl]phenyl acetic acid;

methyl-3-[[3-(1-acetoxyhexyl)phenoxy]methyl]benzoate;

methyl-3-[[3-(1-methoxyhexyl)phenoxy]methyl]benzoate;

methyl-3-[[3-[1-(tetrahydro-2H-pyran-2-yloxy)-hexyl]phenoxy] methyl]benzoate;

methyl-2-[[3-[1-(tetrahydro-2H-pyran-2-yloxy)-pentyl] phenoxy]methyl]benzoate;

2-[3-(1-hydroxypentyl)phenoxy]methyl]benzenemethanol;

3-[[3-(1-hydroxyhexyl)phenoxy]methyl]benzaldehyde;

phenyl-3-formylbenzyl ether;

3-[[3-(1-hydroxyhexyl)phenoxy]methyl]benzyl amine hydrochloride;

3-[[3-(1-hydroxyhexyl)phenoxy]methyl]benzamide;

3-[3-hexanoyl phenoxy]methyl benzamide;

benzyl-3-(hexanoyl)phenyl ether;

3-[3-hexanoyl phenoxy]methyl benzonitrile;

benzyl-3-[1-(N-methylamino)hexyl]phenyl ether;

3-chlorobenzyl-3-[1-(n-butylamino)hexyl]phenyl ether;

2-trifluoromethyl-3-[1-(N,N-dimethylamino)hexyl]-phenyl ether;

benzyl-3-[1-hydroxy-2,2-(dimethyl)hexyl]phenyl ether;

2,4-dibromobenzyl-3-[1-(hydroxy)2-(isobutyl)hexyl] phenyl ether;

4-fluorobenzyl-3-[1-(hydroxy)2-(diethyl)heptyl]-phenyl ether;

benzyl 3-(1-hydroxy-2-methyl hexyl) phenyl ether;

3-(1-hydroxyhexyl)benzyl alcohol;

tolyl-3-(1-hydroxyhexyl)genzyl sulfonate;

methyl-3-[[3-(1-hydroxyhexyl)anilinyl]methyl]benzoate;

ethyl-2-[[3-(1-hydroxybutyl)anilinyl]methyl]benzoate;

isopropyl-4-[[3-(1-hydroxybutyl)anilinyl]methyl]-benzoate;

methyl-3-[[2-(1-hydroxyhexyl)anilinyl]methyl]benzoate;

phenyl-3-[1-(hydroxyhexyl]benzyl thioether;

3-methoxyphenyl-3-[1-(hydroxy)hexyl]benzyl thioether;

4-nitrophenyl-3-[1-(hydroxy)hexyl]benzyl thioether;

methyl-2-[[3-(1-hydroxyhexyl)thiophenoxy]methyl]-benzoate;

methyl 3-[3-(1-hydroxyhexyl) phenyl)amino]carbonyl] benzoate;

2-(3-(1-hydroxy-6,6,6-trifluorohexyl)phenoxymethyl)-1,4-naphthaquinone;

1-(3-(1-hydroxy-1-methylhexyl)phenoxymethyl)-2-methoxy naphthalene;

1-(3-(6-phenoxy-1-hydroxyhexyl)phenoxymethyl)-4-methoxy naphthalene

2-(1-(3-(1-hydroxy-2,2-dimethylhexyl)phenoxy)-ethyl) naphthalene;

9-(3-(1-hydroxyhexyl)phenoxymethyl)anthracene;

9-(3-(1-hydroxyhexyl)phenoxymethyl)phenanthrene;

3-(3-(1-hydroxyethyl)phenoxy)methyl benzoic acid;

3-(2-phenylethyl) hexanophenone;

methyl 3-(3-(1-hydroxyethyl)phenoxy)methyl benzoate;

3-hexanoyl diphenyl acetylene;

cis-3-(1-hydroxyhexyl) stilbene;

1-(3-(2-phenylethyl)phenyl-1-hexanol;

3-(1-hydroxyhexyl)diphenylacetylene;

trans-3-(1'hydroxyhexyl) stilbene;

trans-3-hexanoyl stilbene;

methyl 3-[3-[1-hydroxy-2,2-dimethylhexyl]phenoxy]-methyl]benzoate;

cis-3-hexanoyl stilbene;

2-(3-hexanoylphenoxy) methyl naphthalene;

methyl 3-[3-(1-hydroxyhexyl) phenyl)amino]-carbonyl] benzoate;

3-(2-quinolinylmethoxy)acetophenone;

2-(3-(1-hydroxy-5,5,5-trifluoropentyl)phenoxymethyl)-quinoline;

2-[3-(6-phenoxy-1-hydroxy-1-methylhexyl)phenoxymethyl]quinoline;

2-[3-(6-(3-chlorophenoxy)-1-hydroxy-1-methylhexyl)-phenoxymethyl]quinoline;

2-[3-(6-(3-trifluoromethylphenoxy)-1-hydroxy-1-methylhexyl)phenoxymethyl]quinoline;

3-[6-phenoxy-1-hydroxyhexyl]phenol;

3-[6-(3-trifluoromethylphenoxy)-1-hydroxyhexyl]phenol;

3-[6-(3-chlorophenoxy)-1-hydroxyhexyl]phenol;

2-[3-(6-(3-chlorophenoxy)-1-hydroxyhexyl)phenoxymethyl]quinoline;

2-[3-(6-phenoxy)-3-1-hydroxyhexyl)phenoxymethyl]-quinoline;

2-[3-(6-(3-trifluoromethylphenoxy)-1-hydroxyhexyl)-phenoxymethyl]quinoline;

2-[3-(1-acetoxyhexyl)phenoxymethyl]quinoline;

2-[3-(1-methoxyhexyl)phenoxymethyl]quinoline;

1-(2-thienyl)-2-(3-hexanoylphenyl)acetylene;

1-(2-thienyl)-2-[3-(1-hydroxyhexyl)phenyl]acetylene;

1-(2-quinolyl)-2-[3-(1-hydroxyhexyl)phenyl]acetylene;

1-(2-indolyl)-2-[3-(1-hydroxyhexyl)phenyl]acetylene;

1-(2-furyl)-2-[3-(1-hydroxyhexyl)phenyl]acetylene;

1-(2-thienyl)-2-[3-(1-hydroxyhexyl)phenyl]ethylene;

1-(2-quinolyl)-2-[3-(1-hydroxyhexyl)phenyl]ethylene;

1-(2-indolyl)-2-[3-(1-hydroxyhexyl)phenyl]ethylene;

1-(2-furyl)-2-[3-(1-hydroxyhexyl)phenyl]ethylene;

1-(2-thienyl)-2-(3-hexanoylphenyl)ethylene;
N-[3-(1-hydroxyhexyl)phenyl]pyridine-2-carboxamide;
2-[3-(1-hydroxyhexylbenzylamino]pyridine;
2-[3-(1-hydroxybutyl)anilinylmethyl]thiophene;
4-[3-(1-hydroxybutyl)anilinylmethyl]quinoline;
2-[2-(1-hydroxyhexyl)anilinylmethyl]quinoline;
3-methoxyphenyl-3-[1-(hydroxy)hexyl]benzyl thioether;
4-nitrophenyl-3-[1-(hydroxy)hexyl]benzyl thioether;
methyl-2-[[3-(1-hydroxyhexyl)thiophenoxy]methyl]-benzoate;
2-(3-hexanoylphenoxymethyl)quinoline;
2-[2-(1-hydroxyhexyl)-5-pyridyloxy]quinoline;
4-[2-(1-hydroxyhexyl)-5-thienylmethoxy]quinoline;
2-[5-(1-hydroxyhexyl)-2-furylmethoxy]pyridine;
2-[5-(1-hydroxyhexyl)-2-pyridyloxymethyl]indole;
2-[4-(1-hydroxyhexyl)phenoxymethyl]pyrrole;

WHAT IS CLAIMED IS

1. A compound of the formula:

$$Ar_1 - X - Ar - Z - (R)_{n'}$$

and the pharmaceutically acceptable salts thereof, wherein

$Ar_1$ is a nitrogen, sulfur or oxygen heterocyclic ring or an aromatic ring;

Ar is a phenyl ring or a nitrogen, oxygen or sulfur heterocyclic ring;

Ar and $Ar_1$ may be fully substituted or less than fully substituted with H, $CH_3$, lower alkyl, aryl, aralkyl, halo, hydroxy, lower alkoxy, $CF_3$, carboxy, alkylcarboxy, arylcarboxy, alkylcarbalkoxy, alkanoyl, formyl, oxo, nitrilo, amino, aminoalkyl, alkylamine, carboxamide, aryloxy, nitro, sulfonyl, sulfonamide, thio, alkylthio, hydroxyalkyl or oxyalkylcarboxy;

X = $-O(CHR_1)_n-$, $-\underset{\underset{(O)_{n''}}{\|}}{S}(CHR_1)_n-$, $-NR_2(CHR_1)_n-$alkylene of up to 2 carbon atoms in the principal chain and up to a total of 4 carbon atoms, $-C(R_1)=C(R_1)-$, $-C\equiv C-$, $-\underset{\underset{O}{\|}}{C}(CHR_1)_n-$, $-\underset{\underset{OH}{|}}{CH}(CHR_1)_n-$, $-CH=N-$, $-\underset{\underset{O}{\|}}{C}-O-$, $-\underset{\underset{O}{\|}}{C}-S-$, or $-\underset{\underset{O}{\|}}{C}-N(R_1)-$;

Z is an alkylene chain containing up to 10 carbon atoms in the principal chain and a total of up to 12 carbon atoms and from 0 to 2 double bonds and the said alkylene chain may be attached to Ar through an oxygen, sulfur or amino nitrogen atom, and when $n' \geq 2$, one of the R substituents may be halogen on an omega carbon of the alkylene chain Z;

when $n'=1$, R is a substituent attached to one of the carbon atoms of Z selected from the group consisting of =O, $OR_3$, $SR_3$, $N(R_2)_2$, $R_1$, and $-COR_4$ and when $n' \geq 2$ one R is as previously defined, and the additional R is a substituent attached to one of the carbon atoms of Z selected from the group consisting of =O, $OR_3$, $SR_3$, $N(R_2)_2$, $-COR_4$, lactone and halo;

$R_1$ is H or $CH_3$;

$R_2$ is H, lower alkyl, aryl or aralkyl;

$R_3$ is H, lower alkyl, lower alkanoyl, aryl, aralkyl or substituted aryl in which the substituent is halo, lower alkyl or lower alkoxy;

$R_4$ is $OR_2$ or $N(R_2)_2$;

n = 0 or 1;

n' = 1 to 7; and

n" = 0, 1 or 2.

2. A compound of claim 1 of the formula:

$$Ar_1 - X - Ar - Z - (R)_{n'}$$

and salts thereof,

wherein

$Ar_1$ is quinolyl;

Ar is a phenyl, pyridyl or quinolyl ring;

X = $-O(CHR_1)_n-$, $-\underset{(O)_{n''}}{S}(CHR_1)_n-$, $NR_2(CHR_1)_n-$, alkylene of up to 2 carbon atoms in the principal chain and up to a total of 4 carbon atoms, $-C(R_1)=C(R_1)-$, $-C{\equiv}C-$, $-\underset{O}{\overset{\|}{C}}(CHR_1)_n-$, $-\underset{OH}{CH}(CHR_1)_n-$, $-CH=N-$, $-\underset{O}{\overset{\|}{C}}-O-$, $-\underset{O}{\overset{\|}{C}}-S-$, $-\underset{O}{\overset{\|}{C}}-N(R_1)-$;

Z is an alkylene chain containing up to 10 carbon atoms in the principal chain and a total of up to 12 carbon atoms and from 0 to 2 double bonds;

each R is a substituent, attached to one of the carbon atoms of Z selected from the group consisting of =O, $OR_3$, $SR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl;

$R_4$ is $OR_2$ or $N(R_2)_2$;

n = 0 or 1;

n' = 1 to 7; and

n" = 0, 1 or 2.

3. A compound of claim 1 of the formula:

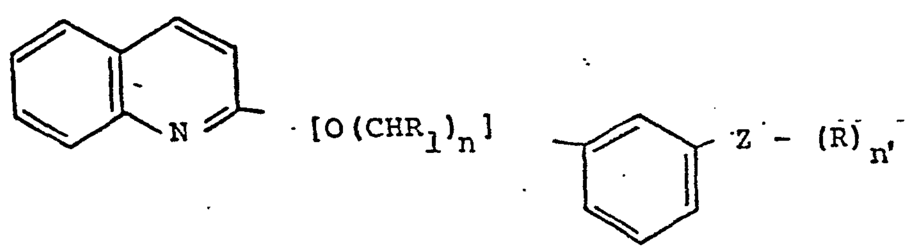

and salts thereof,

wherein

Z is an alkylene chain containing up to 10 carbon atoms in the principal chain and a total of up to 12 carbon atoms and from 0 to 2 double bonds;

n = 0 or 1;

n' = 1 to 7; and

Each R is a substituent, attached to one of the carbon atoms of Z selected from the group consisting of =O, $OR_3$, $SR_3$ $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl;

$R_4$ is OH, $OR_2$ or $N(R_2)_2$;

n = 0 or 1;

n' = 1 to 7;

with the proviso that one R is on the carbon atom of Z attached to the phenyl group.

4. The compound according to Claim 1 which is 2-[3-(1-methyl-1-hydroxyhexyl)phenoxymethyl]quinoline, 2-[3-(2,2-dimethyl-1-hydroxyhexyl)phenoxymethyl]pyridine, 2-[3-(1,2,2-trimethyl-1-hydroxyhexyl)phenoxymethyl]quinoline, 2-[3-(1-methyl-1-hydroxyhexyl)phenoxymethyl]pyridine, 2-[3-(6-(6-[3-chlorophenoxyl]-1-hydroxyhexyl)phenoxymethyl]quinoline or 2-[3-(6-phenoxy-1-hydroxyhexyl)-phenoxymethyl]quinoline.

5. The compound according to Claim 1 which is 2-[3-(6-phenoxy-2,2-dimethyl-1-hydroxyhexyl)phenoxymethyl]quinoline, 2-[3-(6-[3-trifluoromethylphenoxy]-1-hydroxyhexyl)phenoxymethyl]quinoline, 2-[6-(1-hydroxyhexyl)-3-pyridyloxy]quinoline, 2-[3-(6-[3-chlorophenoxy]-1-methyl-1-hydroxyhexyl)phenoxymethyl]quinoline, 2-(3-(1-hydroxy-5,5,5-trifluoropentyl)phenoxymethyl)-quinoline or 2-(3-(1-hydroxy-5,5,5-trifluoropentyl)-phenoxymethylpyridine.

6. The compound according to Claim 1 which is 2-(3-(1-hydroxy-6,6,6-trifluorohexyl)phenoxymethyl)-quinoline, 2-(3-(1-hydroxy-6,6,6-trifluorohexyl)phenoxymethyl)pyridine, 2-(3-(1-hydroxyheptyl)phenoxymethyl)-quinoline, 2-(3-(1-hydroxyheptyl)phenoxymethyl)pyridine, 4-(3-(1-hydroxy-5,5,5-trifluoropentyl)phenoxymethyl)-quinoline, 4-(3-(6-phenoxy-1-hydroxyhexyl)phenoxymethyl quinoline or 2-(3-(6-phenoxy-1-hydroxyhexyl)phenoxymethyl) pyridine.

7. The compound according to Claim 1 which is 2-(3-(1-hydroxy-1-methyl-5,5,5-trifluoropentyl)-phenoxymethyl)quinoline, 2-(3-(1-hydroxy-2,2-dimethyl-5,5,5-trifluoropentyl)-phenoxymethyl)quinoline, 2-(3-(1,2,2-trimethyl-5,5,5-trifluoro-1-hydroxypentyl)-phenoxymethyl)quinoline, 2-(3-(1-methyl-1-hydroxy-5,5,5-trifluoropentyl)-phenoxymethyl)quinoline, 2-(3-(2,2-dimethyl-6,6,6-trifluoro-1-hydroxyhexyl)phenoxymethyl)-quinoline, 2-(3-(1,2,2-trimethyl-6,6,6-trifluoro-1-hydroxyhexyl)phenoxymethyl)quinoline, 2-(3-(2,2-dimethyl-6,6,6-trifluoro-1-hydroxyhexyl)-phenoxymethyl)pyridine, 3-(3-(2,2-dimethyl-6,6,6-trifluoro-1-hydroxyhexyl)-phenoxymethyl)pyridine, 4-(3-(2,2-dimethyl-1-1-hydroxyhexyl)phenoxymethyl)quinoline or 2-(3-(6-methoxyhexyl)-phenoxymethyl)quinoline.

8. The compound according to Claim 1 which is 2-(3-(4-methoxybutyl)phonoxymethyl)quinoline, 2-(4-(4-methoxybutyl)phenoxymethyl)quinoline, 2-(3-(6-phenoxy-hexyl)phenoxymethyl)quinoline, 2-(3-(4-methoxybutyl)-phenoxy)quinoline, 2-(3-(1-acetoxyhexyl)phenoxymethyl)-quinoline, 2-(3-1-(1,1,1-trimethylacetoxy)hexyl)phenoxy-methyl)quinoline, 2-(3-(1-acetoxy-5,5,5-trifluoropentyl)-phenoxymethylquinoline, 2-(3-1-(1,1,1-(trimethylacetoxy)-5,5,5-trifluoropentyl)phenoxymethyl)quinoline,

2-(3-1-(1,1,1-(trimethylacetoxy)-6,6,6-trifluorohexyl)-phenoxymethyl)quinoline, 2-(3-(2,2-dimethyl-1-(1,1,1-trimethylacetoxy)hexylphenoxymethyl)quinoline, 2-(3-(2,2-dimethyl-6,6,6-trifluoro-1-(1,1,1-trimethylacetoxy)-hexyl)phenoxymethyl)quinoline or ethyl 2-(3-(2-quinolinylmethoxy)phenoxy)propionate.

9. The compound according to Claim 1 which is 2-(3-(2-quinolinylmethoxy)phonoxy)propionic acid, ethyl 2-(3-(2-quinolinylmethoxy)phenyl)propionate, 2-(3-(2-quinolinylmethoxy)phenyl)propionic acid, 3-(3-(1-hydroxyhexyl)phenoxymethyl)quinoline, 6-(3-(1-hydroxyhexyl)phenoxymethyl)quinoline, 2-(3-(1-hydroxyhexyl)benzyloxymethyl)quinoline, 2-[3-(6(3-chlorophenoxy)-1-hydroxyhexyl)phenoxymethyl]quinoline, 2-[(3-(6-phenoxy)-1-hydroxyhexyl)phenoxymethyl]quinoline, 2-(3-(6(3-trifluoromethylphenoxy)-1-hydroxyhexyl)-phenoxymethyl]quinoline, 3-[6-(3-chlorophenoxy)-1-hydroxyhexyl]-phenol, 3-[6-phenoxy-1-hydroxyhexyl]phenol, 3-[6-(3-trifluoromethylphenoxy)-1-hydroxyhexyl]phenol, 2-[3-(6-phenoxy-1-hydroxy-1-methylhexyl)phenoxymethyl]-quinoline or 2-[3-(6-(3-chlorophenoxy)-1-hydroxy-1-methylhexyl)phenoxymethyl]quinoline.

10. The compound according to Claim 1 which is 2-[3-(6-(3-trifluoromethylphenoxy)-1-hydroxy-1-methylhexyl)phenoxymethyl]quinoline, 3-(2-quinolinylmethoxy) acetophenone, 2-[3-(1-Hydroxyhexyl)phenoxymethyl]quinoline, (2-quinolinylmethoxy)phenylacetic acid, 2-(3-(6-phenoxy-1,2,2-trimethyl-1-hydroxyhexyl)phenoxymethyl)quinoline, 2-(3-hexylphenoxy)methyl quinoline, 1-(3-(2-quinolinylmethoxy)phenyl)-3-methyl-1,3-butane diol, 2-(3-(6,6,6-trifluorohexanoyl)phenoxymethyl)quinoline, 2-(3-(5,5,5-trifluoropentanoyl)phenoxymethyl)quinoline, 2-(3-(6-benzyloxy-1-hydroxyhexyl)phenoxymethyl)quinoline, methyl(3-(3-(2-quinolinylmethoxy)benzoyl)propionoate or 2-[3-(6-phenoxy-1-hydroxyhexyl)phenoxymethyl]quinoline.

11. A compound of Claim 1 of the formula:

$Ar_1 - X - Ar - Z - (R)_{n'}$,

and salts thereof,

wherein

$Ar_1$ is a phenyl or naphthyl group;

Ar is a phenyl, pyridyl or quinolyl group;

$X = -O(CHR_1)_n-$, $-\underset{(O)_{n''}}{\overset{}{S}}(CHR_1)_n-$, $NR_2(CHR_1)_n-$, alkylene of up to 2 carbon atoms in the principal chain and up to a total of 4 carbon atoms, $-C(R_1)=C(R_1)-$, $-C{\equiv}C-$, $-\underset{O}{\overset{}{C}}(CHR_1)_n-$, $-\underset{OH}{CH}(CHR_1)_n-$, $-CH=N-$, $-\underset{O}{C}-O-$, $-\underset{O}{C}-S-$, $-\underset{O}{C}-N(R_1)-$;

Z is an alkylene chain containing up to 10 carbon atoms in the principal chain and a total of up to 12 carbon atoms and from 0 to 2 double bonds

each R is a substituent, attached to one of the carbon atoms of Z selected from the group consisting of =O, $OR_3$, $SR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl or an O-heterocyclic group;

$R_4$ is $OR_2$ or $N(R_2)_2$;

n = 0 or 1;

n' = 1 to 7; and

n" = 0, 1 or 2.

12. A compound of Claim 1 of the formula:

$Ar_1 - X - Ar - Z - (R)_{n'}$

and salts thereof;

wherein

$Ar_1$ is a phenyl or naphthyl group;

Ar is a phenyl group;

$X = -O(CHR_1)_n-$, $-\underset{\underset{(O)_{n''}}{\|}}{S}(CHR_1)_n-$, $-NR_2(CHR_1)_n-$, alkylene of up to 2 carbon atoms in the principal chain and up to a total of 4 carbon atoms; $-C(R_1)=C(R_1)-$, $-C\equiv C-$, $-CH=N-$, $-\underset{\underset{O}{\|}}{C}-N(R_1)-$;

Z is alkylene chain containing up to 10 cabon atoms in the principal chain and a total of up to 12 carbon atoms and from 0 to 2 double bonds and the

each R is a substituent, attached to one of the carbon atoms of Z selected from the group consisting of =O, $OR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl and said O-heterocyclic group is a pyran group or tetrahydropyran group;

$R_4$ is $OR_2$ or $N(R_2)_2$;

n = 0 or 1;

n' = 1 to 7; and

n" = 0, 1 or 2.

13. The compound according to Claim 1 which is methyl 3-[[3-(1-hydroxyhexyl)phenoxy]methyl]benzoate, methyl 2-[[3-(1-hydroxyhexyl)phenoxy]methyl]benzoate, 3[[3-(1-hydroxyhexyl)phenoxy[methyl[benzamide, methyl-3-[3-(1-methyl-1-hexenyl)phenoxy]methy]benzoate, 1-(3-phenoxyphenyl)-1-hexanol, 1-(3-benzyloxphenyl)-1-methyl-1-pentanol, 2-(3-hexanoylphenoxy)methyl naphthalene, 2-(3-(1-hydroxyhexyl)phenoxymethyl)naphthalene or 2-(3-(1-hydroxyhexyl)-phenoxymethyl)-1,2,3,4-tetrahydronaphthalene.

14. A method of treating inflammatory and allergic conditions in a mammal by administering to said mammal an effective amount of a compound of any of Claims 1-13.

15. A process for the production of a compound as in Claim 1, which includes at least one of the following:

(a) condensation of a compound of formula II and III herein in which X' and X" are $-CH_2Hal$, Hal, OH, $CH_2OH$, SH or $CH_2SH$, the nature of X' and X" being such to permit formation of Z on condensing to a compound of formula I herein;

(b) condensation of compounds of formula II and III herein in which X' and X" are $-CH=CH_2-$, $-C\equiv CH$, or Hal, the nature of X' and X" being such to permit formation of Z on condensing to a compound of formula I herein;

(c) condensing compounds of formulas II and III herein in which X' and X" are -CHO or $-NH_2-$, the nature of X' and X" being such to permit formation of Z on condensing to a compound of formula I herein;

(d) condensing compounds of formula II and III herein in which X' and X" are COOH, or NH, the nature of X' and X" being such to permit formation of Z on condensing to a compound of formula I herein;

16. The process of Claim 15 where the compounds of Formula I are:

$Ar_1 - X - Ar - Z - (R)_{n'}$,

and salts thereof,

wherein

$Ar_1$ is quinolyl;

Ar is a phenyl, pyridyl or quinolyl ring

$X = -O(CHR_1)_n-$, $-\underset{(O)_{n''}}{\overset{}{S}}(CHR_1)_n-$, $NR_2(CHR_1)_n-$; alkylene of up to 2 carbon atoms in the principal chain and up to a total of 4 carbon atoms, $-C(R_1)=C(R_1)-$, $-C\equiv C-$, $-(\underset{O}{\overset{}{C}}HR_1)_n-$, $-\underset{OH}{CH}(CHR_1)_n-$, $-CH=N-$, $-\underset{O}{C}-O-$, $-\underset{O}{C}-S-$, $-\underset{O}{C}-N(R_1)-$;

Z is an alkylene chain containing up to 10 carbon atoms in the principal chain and a total of up to 12 carbon atoms and from 0 to 2 double bonds

each R is a substituent, attached to one of the carbon atoms of Z selected from the group consisting of $=O$, $OR_3$, $SR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl;

$R_4$ is $OR_2$ or $N(R_2)_2$;

n = 0 or 1;

n' = 1 to 7; and

n" = 0, 1 or 2.

17. The process of Claim 15 where the compounds of Formula I are:

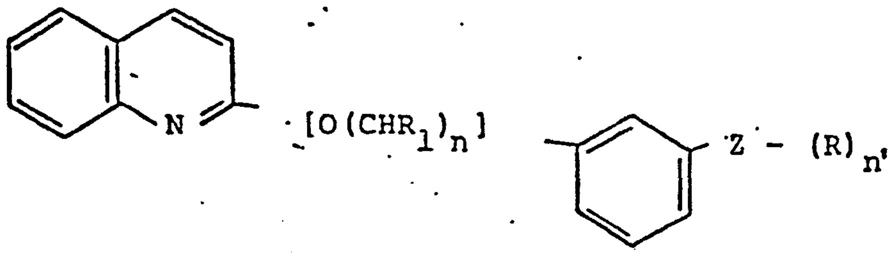

and salts thereof,

wherein

Z is an alkylene chain containing up to 10 carbon atoms in the principal chain and a total of up to 12 carbon atoms and from 0 to 2 double bonds;

n = 0 or 1;

n' = 1 to 7; and

Each R is a substituent, attached to one of the carbon atoms of Z selected from the group consisting of =O, $OR_3$, $SR_3$ $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl;

$R_4$ is OH, $OR_2$ or $N(R_2)_2$;

n = 0 or 1;

n' = 1 to 7;

with the proviso that one R is on the carbon atom of Z attached to the phenyl group.

18. The process of Claim 15 where the compounds of Formula I are:

$$Ar_1 - X - Ar - Z - (R)_{n'}$$

and salts thereof,

wherein

$Ar_1$ is a phenyl or naphthyl group;

Ar is a phenyl, pyridyl or quinolyl group;

$X = -O(CHR_1)_n-$, $-\underset{(O)_{n''}}{S}(CHR_1)_n-$, $NR_2(CHR_1)_n-$, alkylene of up to 2 carbon atoms in the principal chain and up to a total of 4 carbon atoms, $-C(R_1)=C(R_1)-$, $-C{\equiv}C-$, $-\underset{\underset{O}{\|}}{C}(CHR_1)_n-$, $-\underset{\underset{OH}{|}}{CH}(CHR_1)_n-$, $-CH=N-$, $-\underset{\underset{O}{\|}}{C}-O-$, $-\underset{\underset{O}{\|}}{C}-S-$, $-\underset{\underset{O}{\|}}{C}-N(R_1)-$;

Z is an alkylene chain containing up to 10 carbon atoms in the principal chain and a total of up to 12 carbon atoms and from 0 to 2 double bonds

each R is a substituent, attached to one of the carbon atoms of Z selected from the group consisting of =O, $OR_3$, $SR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl or an O-heterocyclic group;

$R_4$ is $OR_2$ or $N(R_2)_2$;

n = 0 or 1;

n' = 1 to 7; and

n" = 0, 1 or 2.

19. The process of Claim 15 wherein the compounds of Formula I are:

$$Ar_1 - X - Ar - Z - (R)_{n'}$$

and salts thereof;

wherein

$Ar_1$ is a phenyl or naphthyl group;

Ar is a phenyl group;

$X = -O(CHR_1)_n-$, $-\underset{(O)_{n''}}{\overset{\|}{S}}(CHR_1)_n-$, $-NR_2(CHR_1)_n-$, alkylene of up to 2 carbon atoms in the principal chain and up to a total of 4 carbon atoms; $-C(R_1)=C(R_1)-$, $-C{\equiv}C-$, $-CH=N-$, $-\underset{O}{\overset{\|}{C}}-N(R_1)-$;

Z is alkylene chain containing up to 10 cabon atoms in the principal chain and a total of up to 12 carbon atoms and from 0 to 2 double bonds and the

each R is a substituent, attached to one of the carbon atoms of Z selected from the group consisting of =O, $OR_3$, $N(R_2)_2$ and $-COR_4$;

$R_1$ is H or $CH_3$;

$R_2$ is H, lower alkyl, aryl or aralkyl;

$R_3$ is H, lower alkyl, lower alkanoyl, aryl or aralkyl and said O-heterocyclic group is a pyran group or tetrahydropyran group;

$R_4$ is $OR_2$ or $N(R_2)_2$;

n = 0 or 1;

n' = 1 to 7; and

n" = 0, 1 or 2.